# EUROPEAN PATENT APPLICATION

(11) **EP 4 775 579 A1**
(43) Date of publication of application: **15.07.2026**
(21) Application number: 24862932.1
(22) Date of filing: 09.09.2024
(51) Int. Cl.: C07D 455/03, A01N 43/90, A01P 21/00, A23L 33/105, A61K 8/49, A61K 31/4745, A61P 3/00, A61P 3/04, A61P 15/08, A61P 17/16, A61P 21/00, A61P 25/16, A61P 25/28, A61P 35/00, A61Q 19/08

(54) **BERBERRUBINE-RELATED COMPOUND**

(30) Priority: 07.09.2023 JP 2023144899
(71) Applicant: Mitogenic Co., Ltd., Tokyo 169-0075 (JP)
(72) Inventor: YANAGI, Shigeru, Tokyo 171-8588 (JP); ABE, Hideki, Tokyo 112-8681 (JP); TANIWAKA, Keito, Tokyo 169-0075 (JP); SHIIBA, Isshin, Tokyo 171-8588 (JP); TANABU, Daishi, Tokyo 171-8588 (JP); OIKE, Yuichi, Kumamoto-shi, Kumamoto 860-8556 (JP); TAKASHIMA, Akihiko, Tokyo 171-8588 (JP); SOEDA, Yoshiyuki, Tokyo 171-8588 (JP); HARADA, Hironori, Hachioji-shi, Tokyo 192-0392 (JP); INOUE, Satoshi, Tokyo 173-0015 (JP); OKAZAKI, Yasushi, Tokyo 113-8421 (JP); SUGIURA, Ayumu, Tokyo 113-8421 (JP); OKADA, Futoshi, Yonago-shi, Tottori 683-8503 (JP)
(74) Representative: Latscha Schöllhorn Partner AG
(86) International application number: PCT/JP2024/032131
(87) International publication number: WO 2025/053283

(57) **Abstract**

Provided are: a berberrubine-related compound having useful physiological activities; and a composition containing the berberrubine-related compound. The composition contains quinoid-type berberrubine. Also provided are a berberrubine acetic acid adduct and an aqueous solution thereof. Also provided is a composition containing a berberrubine acetic acid adduct or an aqueous solution thereof. Also provided is a method for producing a berberrubine acetic acid adduct or an aqueous solution thereof. Also provided are a composition containing quinoid-type berberrubine and a composition containing a berberrubine acetic acid adduct or an aqueous solution thereof, each of which can be used as a pharmaceutical composition, a food composition, a cosmetic composition, or an agricultural composition.

## Description

### TECHNICAL FIELD

The present disclosure relates to berberrubine-related compounds, and in particular, a composition containing quinoid-type berberrubine, a berberrubine acetic acid adduct, and a method for producing the same, as well as a composition containing the berberrubine acetic acid.

### BACKGROUND

Berberrubine is a derivative of berberine, which is an alkaloid found in Coptis japonica, Phellodendron amurense, etc. Berberine is an ingredient in herbal medicines that have stomachic and anti-inflammatory effects, and tranquilizing effects, and berberrubine is believed to be a metabolite of berberine in the body.

Non-Patent Document 1 describes that berberrubine can take the form of (i) a quinoid-type, (ii) an enol-type, or (iii) a zwitterionic-type.

Non-Patent Document 1: Spinozzi, S. et al. Journal of Natural Products, 77, 766-722, 2014

### SUMMARY OF INVENTION

The present disclosure provides berberrubine-related compounds having useful physiological activity and compositions containing the same.

The present inventors discovered, surprisingly, that compositions containing quinoid-type berberrubine have useful physiological activity, that a berberrubine acetic acid adduct can be obtained by adding acetic acid to quinoid-type berberrubine, and that a composition containing a berberrubine acetic acid adduct has high water solubility and also useful physiological activity, etc., thereby completing the present invention.
That is, according to the present invention, the following aspects are provided.
[1] A composition containing quinoid-type berberrubine represented by the following Formula (I). 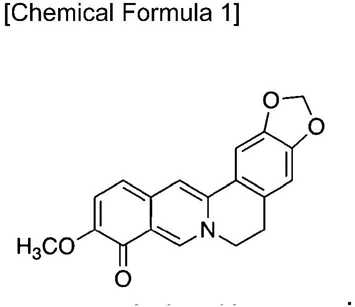
[2] A method for producing a berberrubine acetic acid adduct, the method including: adding acetic acid to quinoid-type berberrubine represented by the following Formula (I). 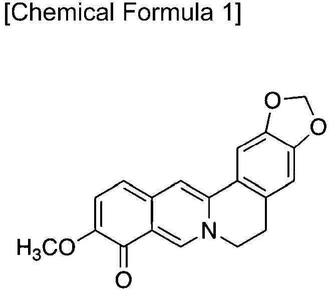
[3] A berberrubine acetic acid adduct.

According to the present invention, berberrubine-related compounds having useful physiological activity and compositions containing the same can be provided.

### BRIEF DESCRIPTION OF DRAWINGS

FIG. 1A shows a solid-state ¹³C NMR spectrum of quinoid-type berberrubine according to the CPMAS method.
FIG. 1B shows a solid-state ¹³C NMR spectrum of quinoid-type berberrubine according to the DDMAS method.
FIG. 2A shows a solid-state ¹³C NMR spectrum of a berberrubine acetic acid adduct according to the CPMAS method.
FIG. 2B shows a solid-state ¹³C NMR spectrum of a berberrubine acetic acid adduct according to the DDMAS method.
FIG. 3A shows a solid-state ¹³C NMR spectrum of a berberrubine acetate salt according to the CPMAS method.
FIG. 3B shows a solid-state ¹³C NMR spectrum of a berberrubine acetate salt according to the DDMAS method.
FIG. 4A shows the change in oxygen consumption rate (OCR) over time in a C2C12 cell to which quinoid-type berberrubine has been added. The point at which 24 hours had passed after addition was set to 0 hour (minutes). At the points of 20, 40, and 70 minutes (shown by black arrows), an ATP synthase inhibitor (oligomycin), an uncoupling agent (carbonylcyanide-m-chlorophenylhydrazone (CCCP)), and mitochondrial complex inhibitors (rotenone and antimycin) were added, respectively.
FIG. 4B shows the change over time in the oxygen consumption rate (OCR) in a C2C12 cell to which a berberrubine acetic acid adduct has been added. The point at which 24 hours had passed after addition was set to 0 hour (minutes). At the points of 20, 40, and 70 minutes (shown by black arrows), an ATP synthase inhibitor (oligomycin), an uncoupling agent (carbonylcyanide-m-chlorophenylhydrazone (CCCP)), and a mitochondrial complex inhibitor (rotenone and antimycin) were added, respectively.
FIG. 4C shows the OCRs at the points of 30, 60, and 90 minutes (shown by white arrows) (corresponding to the amounts of basal respiration, ATP production, and maximal respiration, respectively, in mitochondria) in FIG. 4A.
FIG. 4D shows the OCRs at the points of 30, 60, and 90 minutes (shown by white arrows) (corresponding to the amounts of basal respiration, ATP production, and maximal respiration, respectively, in mitochondria) in FIG. 4B.
FIG. 5 shows the results of detecting the formation of mitochondrial respiratory chain supercomplexes in C2C12 cells to which quinoid-type berberrubine or a berberrubine acetic acid adduct has been added.
FIG. 6A shows the comparison of the expression levels of mitochondrial proteins OPA1, HSP60, and Mfn2 in primary cultured mouse fetal pituitary cells to which a berberrubine acetic acid adduct was administered, the nuclei having been stained with Hoechst and the mitochondria having been stained with Tom20.
FIG. 6B shows the results of observing, with a fluorescence microscope (magnification: 60x), the primary cultured mouse fetal pituitary cells to which a berberrubine acetic acid adduct was administered, the nuclei having been stained with Hoechst and the mitochondria having been stained with Tom20.

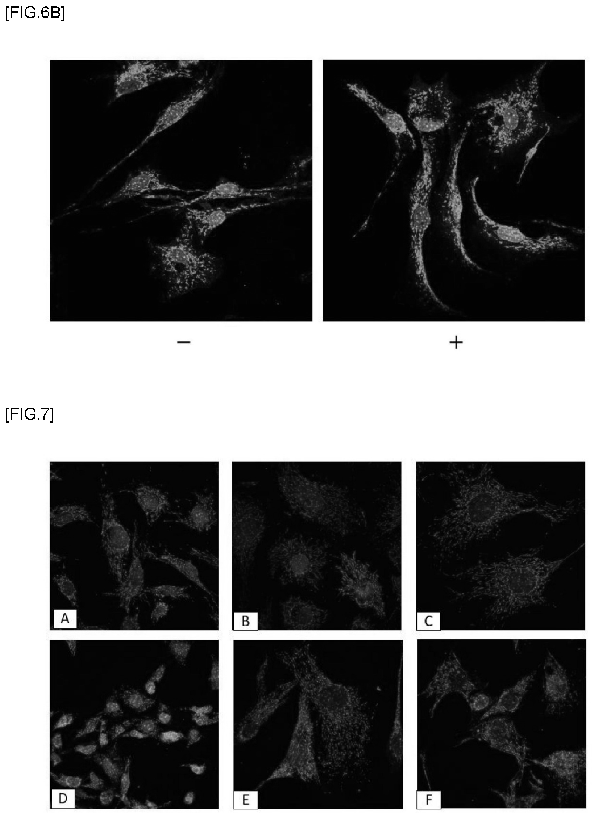

FIG. 7 shows the results of observing, with a fluorescence microscope (magnification: 60x), Pink1 KO MEF cells and MEF cells to which quinoid-type berberrubine or a berberrubine acetic acid adduct was administered, the nuclei having been stained with Hoechst and the mitochondria having been stained with Tom20. A shows wild-type MEF cells (control; to which only DMSO was administered), B shows wild-type MEF cells to which quinoid-type berberrubine was administered, and C shows wild-type MEF cells to which a berberrubine acetic acid adduct was administered. Moreover, D shows MEF_pink1 KO cells (control; to which only DMSO was administered); E shows MEF_pink1 KO cells to which quinoid-type berberrubine was administered, and F shows MEF_pink1 KO cells to which a berberrubine acetic acid adduct was administered.
FIG. 8A shows the ratio of the number of cells of the HeLa cells after being administered quinoid-type berberrubine (quinoid-type berberrubine) with respect to the number of cells of the HeLa cells of the control (to which only DMSO was administered) (control).
FIG. 8B shows the ratio of the number of cells of the HeLa cells after being administered a berberrubine acetic acid adduct (berberrubine acetic acid adduct) with respect to the number of cells of the HeLa cells of the control (to which only water was administered) (control).
FIG. 9 shows the ratio of the number of MDS clones with respect to the number of blood cells in bone marrow transplant (BMT) mice to which a berberrubine acetic acid adduct was administered.
FIG. 10A shows the results of Neuro2A cells with intracellular aggregation of tau proteins, being administered quinoid-type berberrubine, and then detecting the tau proteins by means of Western blotting using an antibody against phosphorylated Tau at position S396 (pS396), an antibody against phosphorylated Tau at position S422 (pS422), and an antibody against full length Tau (JM).
FIG. 10B shows the results of Neuro2A cells with intracellular aggregation of tau proteins, being administered a berberrubine acetic acid adduct, and then detecting the tau proteins by means of Western blotting using an antibody against phosphorylated Tau at position S396 (pS396), an antibody against phosphorylated Tau at position S422 (pS422), and an antibody against full length Tau (JM).
FIG. 10C shows the results of quantification and comparison of the tau proteins detected in FIG. 10A.
FIG. 10D shows the results of quantification and comparison of the tau proteins detected in FIG. 10B.
FIG. 11A is a snapshot of a video, captured with a fluorescence microscope (magnification: 20x), of the movement of mouse spermatozoa to which a berberrubine acetic acid adduct or control was administered, with mitochondria stained.
FIG. 11B is a graph showing the number of spermatozoa confirmed to have mitochondrial staining by MitoTracker in the image in FIG. 11A.
FIG. 11C is a snapshot of a video, captured with an optical micrograph (magnification: 20x), of mouse spermatozoa to which a berberrubine acetic acid adduct or control was administered.
FIG. 12 shows the effect of quinoid-type berberrubine on the skin of mice irradiated with UV. + is the mouse to which quinoid-type berberrubine was administered and - is the mouse to which quinoid-type berberrubine was not administered.
FIG. 13 shows the effect of quinoid-type berberrubine on the accumulation of mouse visceral fat. + is the mouse to which quinoid-type berberrubine was administered and - is the mouse to which quinoid-type berberrubine was not administered.
FIG. 14 shows the results of observing, with a fluorescence microscope (magnification: 60x), brown fat cells to which quinoid-type berberrubine or a berberrubine acetic acid adduct was added and brown fat cells to which quinoid-type berberrubine or a berberrubine acetic acid adduct was not added, the nuclei having been stained with Hoechst and the mitochondria having been stained with TMRM.
FIG. 15A shows the growth of bean sprouts 3 days after being administered an aqueous solution of a berberrubine acetic acid adduct or the same amount of water.
FIG. 15B shows the growth of bean sprouts 9 days after being administered an aqueous solution of a berberrubine acetic acid adduct or the same amount of water.
FIG. 15C shows the growth of bean sprouts 12 days after being administered an aqueous solution of a berberrubine acetic acid adduct or the same amount of water.
FIG. 16 shows echocardiograms of mice and control mice to which a berberrubine acetic acid adduct was administered. Heart rate (HR), blood pressure (BP), left ventricular inflow ratio (E/A), heart rate left ventricular end-diastolic diameter (LVDd), left ventricular end-systolic diameter (LVDs), left ventricular ejection fraction (LVEF), % left ventricular inner diameter fractional shortening (%FS), and left ventricular filling pressure (E/e') are shown. n=7-9, and error bars indicate standard deviation.
FIG. 17 shows the heart weight, heart weight/body weight ratio, and lung weight/body weight ratio of mice to which a berberrubine acetic acid adduct was administered and control mice. n=7-9, and the error bars indicate standard deviation.
FIG. 18 shows the expressions of cardiac failure and myocardial fibrosis markers in mice to which a berberrubine acetic acid adduct was administered and control mice. n= 7-9, and the error bars indicate standard deviation.
FIG. 19 shows the effect of a berberrubine acetic acid adduct on the expression of mitochondrial mRNA. n=6-9, and the error bars indicate standard deviation.
FIG. 20 shows echocardiograms of mice to which a berberrubine acetic acid adduct or quinoid-type berberrubine was administered and control mice. Heart rate left ventricular end-diastolic diameter (LVDd), left ventricular end-systolic diameter (LVDs), left ventricular ejection fraction (LVEF), % left ventricular inner diameter fractional shortening (%FS), and left ventricular filling pressure (E/e') are shown. n=7-9, and the error bars indicate standard deviation. * indicates p<0.05.
FIG. 21 shows the expressions of cardiac failure and myocardial fibrosis markers in mice to which a berberrubine acetic acid adduct or quinoid-type berberrubine was administered and control mice. n=7-9, and the error bars indicate standard deviation. *, **, and *** respectively indicate p<0.05, p<0.01, and p<0.001.
FIG. 22 shows the effect of mice to which a berberrubine acetic acid adduct or quinoid-type berberrubine was administered with respect to Mitol mRNA expression. n=7-9, and the error bars indicate standard deviation. *, **, and *** respectively indicate p<0.05, p<0.01, and p<0.001. n.s. indicates that there is no significant difference.

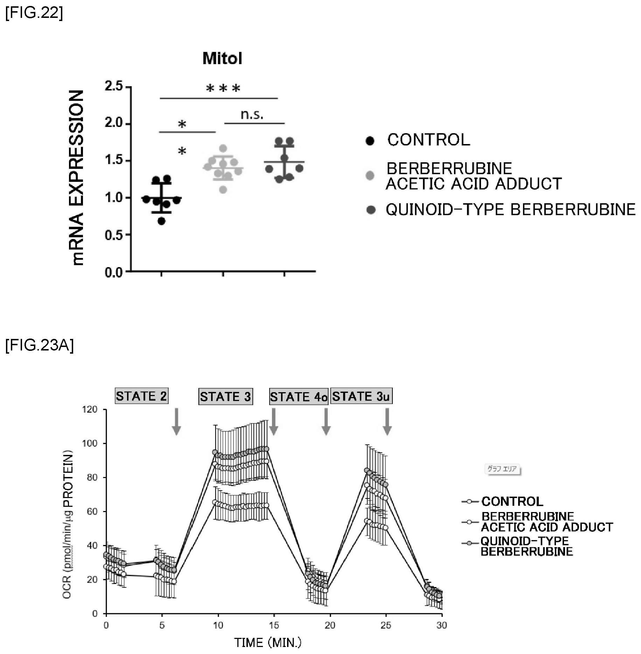

FIG. 23A shows the change over time in the oxygen consumption rate (OCR) of mitochondria isolated from cardiomyocytes of mice to which a berberrubine acetic acid adduct or quinoid-type berberrubine was administered or control mice. n=5, and the error bars indicate standard deviation. The start of the measurement was set at 0 min. ADP was added at 7 min, an enzyme inhibitor (oligomycin) was added at 15 min, a chemical uncoupling agent (carbonyl cyanide-p-trifluoromethoxyphenylhydrazone (FCCP)) was added at 20 min, and mitochondrial complex inhibitors (rotenone and antimycin) was added at 25 min. 0-7 minutes was set as State 2, 7-15 minutes as State 3, 15-20 minutes as State 4o, and 20-25 minutes as State 3u.
FIG. 23B shows the OCRs in State 2, State 3, State 4o, and State 3u of FIG. 23A.

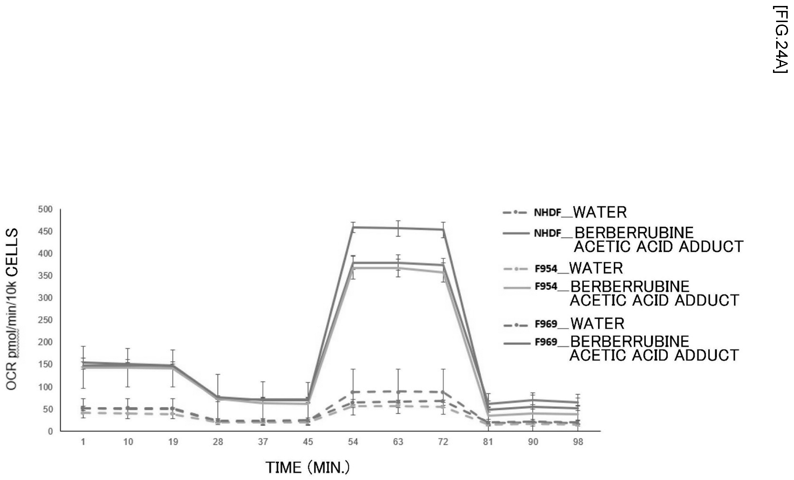

FIG. 24A shows the effect of a berberrubine acetic acid adduct on normal human dermal fibroblast (NHDF) cells and mitochondrial encephalomyopathy, lactic acidosis, and stroke-like episodes (MELAS) derived cells F954 (3243A>G; 92%) and F969 (3243A>G; 34.5%). The experiment started on day 0, water (control) or 10 µM of a berberrubine acetic acid adduct was administered on day 1, and the oxygen consumption rate (OCR) was measured on day 8. The start of the measurement was set at 0 minutes. An enzyme inhibitor (oligomycin) was added at 19 minutes, a chemical uncoupling agent (carbonyl cyanide-p-trifluoromethoxyphenylhydrazone (FCCP)) was added at 45 minutes, and mitochondrial complex inhibitors (rotenone and antimycin) was added at 72 minutes.
FIG. 24B shows the basal respiration (difference between the OCR at 10 minutes and the OCR at 90 minutes), maximal respiration (difference between the OCR at 63 minutes and the OCR at 90 minutes), and reserve respiratory capacity (difference between the OCR at 63 minutes and the OCR at 10 minutes) in FIG. 24A.
FIG. 25 shows the effect of a berberrubine acetic acid adduct in a mouse inflammatory carcinogenesis model.
FIG. 26 shows the survival time and survival rate in a mouse to which a berberrubine acetic acid adduct was administered and control mouse.

### [EXPLANATION OF TERMS]

Unless specified otherwise, the terms used in the present disclosure have the following meanings.

The term "berberrubine" in the present disclosure includes quinoid-type berberrubine, enol-type berberrubine, and zwitterionic-type berberrubine.

The quinoid-type berberrubine has the IUPAC name of 10-methoxy-5,6-dihydro-9H-[1,3]dioxolo[4,5-g]isoquinolino [3,2-a]isoquinolin-9-one and is represented by the following Formula (1) 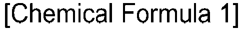 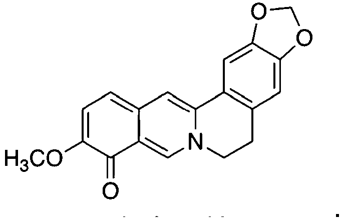 The enol-type berberrubine has the IUPAC name of 5,6-dihydro-9-hydroxy-10-methoxybenzo[g]-1,3-benzodioxolo(5,6-a)quinolizinium and is represented by the following Formula (2) 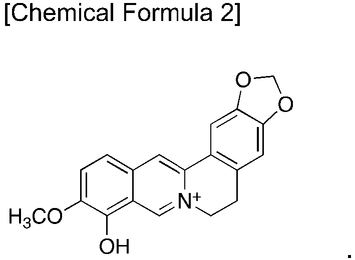 The zwitterionic-type berberrubine has the IUPAC name of 10-methoxy-5,6-dihydro-[1,3]dioxolo[4,5-g]isoquinolino[3,2-a]isoquinolin-7-ium-9-olate and is represented by the following Formula (3).

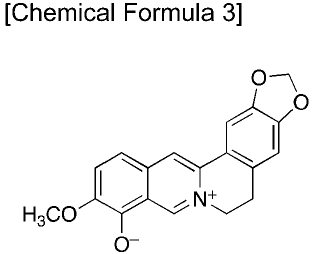

"Berberrubine-related compounds" include berberrubine, berberrubine derivatives, berberrubine precursors, as well as salts, acid adducts, and hydrates thereof.

The "berberrubine acetic acid adduct" in the present disclosure is a compound in which acetic acid and/or acetate ions are added to berberrubine. Within this definition, the equivalent ratio of berberrubine with respect to acetic acid and/or acetate ions is not limited.

In the present disclosure, a berberrubine acetic acid adduct in which 1 equivalent of acetic acid and/or acetate ions are added to 1 equivalent of berberrubine is also referred to as berberrubine acetate salt.

For a given berberrubine acetic acid adduct, when the equivalent amounts of berberrubine, and acetic acid and/or acetate ions are determined based on experimental values, the experimental error is considered to determine whether the berberrubine acetic acid adduct can be considered a berberrubine acetate salt. For example, if the equivalent amounts of berberrubine, and acetic acid and/or acetate ions are determined by ¹³C NMR described below, a berberrubine acetic acid adduct determined to have 1 equivalent of acetic acid and/or acetate ions added to 1 equivalent of berberrubine can be considered a berberrubine acetate salt. Here, the numerals can be interpreted, for example, by applying normal rounding, so a berberrubine acetic acid adduct determined to have 0.5-1.4 equivalents of acetic acid and/or acetate ions added to 1 equivalent of berberrubine can be considered a berberrubine acetate salt, and moreover, a berberrubine acetic acid adduct determined to have 0.9-1.1 equivalents of acetic acid and/or acetate ions added to 1 equivalent of berberrubine may be considered a berberrubine acetate salt.

The berberrubine acetic acid adduct used in the present disclosure is typically,
(i) a berberrubine acetic acid adduct in which acetic acid and/or acetate ions are added to the enol-type berberrubine represented by Formula (2): 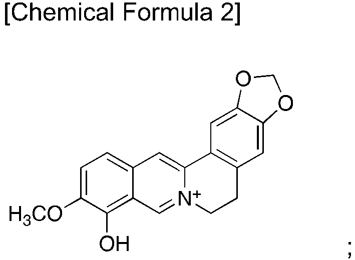
(ii) a berberrubine acetic acid adduct in which acetic acid and/or acetate ions are added to the zwitterionic-type berberrubine represented by Formula (3): 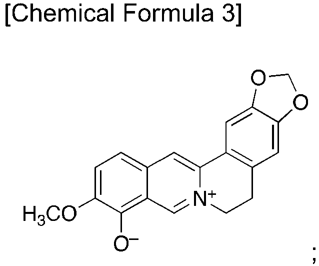 or
(iii) a mixture thereof.

Unless otherwise noted, berberine chloride is used as "berberine" in the present disclosure. Berberine chloride has the IUPAC name of 9,10-Dimethoxy-5,6-dihydro[1,3]dioxolo[4,5-g]isoquinolino[3,2-a]isoquinolin-7-ium chloride.

Berberine chloride is commercially available (e.g., manufactured by NACALAI TESQUE, INC. or TOKYO CHEMICAL INDUSTRY CO., LTD.). Moreover, berberine chloride can also be isolated from nature or synthesized artificially by known methods.

The singular form "a," "an," and "the" as used in the present disclosure may include multiple objects, unless otherwise clearly indicated. As used in the present disclosure, "and/or" includes a relationship represented by "and" and a relationship represented by "or". The term "and" may also be expressed as "moreover" or "as well as". The term "or" may also be expressed as "alternatively". In the present disclosure, "containing" includes "consisting primarily of," "consisting substantially of," and "consisting of," whereas "consisting primarily of" includes "consisting substantially of" and "consisting of," and "consisting substantially of" includes "consisting of".

For each numerical range in the present disclosure, the upper and lower limits indicated by "-" and "to" shall be included, respectively. For example, the descriptions "A-B" or "A to B" using the numerical values A and B means that the value is A or more and B or less. Moreover, the descriptions "A-B," "A to B," or "A or more and B or less" in the numerical ranges described in stages in the present disclosure independently include both "A or more is preferred" and "B or less is preferred", and the lower or upper limits of these values may be replaced by the upper or lower limits of other numerical ranges. Moreover, the lower or upper limits of the numerical ranges described in the present disclosure may be replaced by the numerical values within those numerical ranges shown in the examples.

Unless otherwise specified, it is understood that all figures used herein and in the claims that represent features, items, quantities, parameters, characteristics, periods, etc., are in all cases modified by the term "about". As used herein, the term "about" means that the features, items, quantities, parameters, characteristics, periods, etc. so identified includes a range of plus or minus 10 percent above or below the value of the indicated features, items, quantities, parameters, characteristics, periods, etc. So as not to at least limit the application of the doctrine of equivalents to the claims, each numerical indicator should be at least interpreted by considering the number of reported significant figures and applying normal rounding. Any numerical range or value inherently includes a range of errors that inevitably result from the standard deviations found in their respective test measurements. Unless otherwise specified, each individual value of a numerical range herein is incorporated herein as if it were listed individually herein.

### [Embodiments]

Hereinafter, embodiments of the present disclosure will be described in detail, but the scope of the present invention is not limited to the embodiments described herein, and various modifications can be made without departing from the spirit of the present invention. The embodiments may be implemented alone or in combination. Regarding the definitions and details in the embodiments, see [EXPLANATION OF TERMS] above.

### <Compositions containing quinoidal berberrubine>

In one embodiment, there is provided a composition containing quinoid-type berberrubine represented by the following Formula (I)

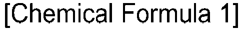

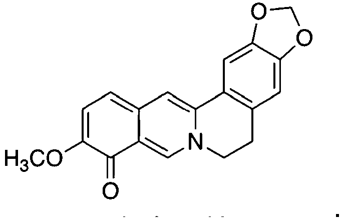

The quinoid-type berberrubine may be isolated quinoid-type berberrubine. Moreover, the quinoid-type berberrubine may be a naturally occurring compound, an artificially synthesized compound, or a compound that is artificially modified from a naturally occurring raw material.

Quinoid-type berberrubine can be obtained, for example, by heating berberine chloride at 190 °C for 3 hours under reduced pressure. Berberine chloride can be isolated from nature or synthesized artificially by known methods and is commercially available (e.g., from NACALAI TESQUE, INC. and TOKYO CHEMICAL INDUSTRY CO., LTD.). Moreover, quinoid-type berberrubine is also commercially available (e.g., from Aurora Fine Chemicals and AMBINTER).

Compositions containing quinoid-type berberrubine may contain quinoid-type berberrubine in any proportion. In an embodiment, the content of quinoid-type berberrubine in a composition containing quinoid-type berberrubine may be 0.00001 mass% or more, 0.0001 mass% or more, 0.001 mass% or more, 0.01 mass% or more, 0.1 mass% or more, 1 mass% or more, 2 mass% or more, 5 mass% or more, 10 mass% or more, 20 mass% or more, 30 mass% or more, 40 mass% or more, 50 mass% or more, 60 mass% or more, 70 mass% or more, 80 mass% or more, 90 mass% or more, 95 mass% or more, 98 mass% or more, 99 mass% or more, or 100 mass%.

Moreover, the composition containing quinoid-type berberrubine may also contain various additives to the extent that they do not impair the effects of the present invention. Non-limiting examples of additives include buffering agents, pH adjusters, surfactants, excipients, preservatives, antiseptics, medicinal ingredients, etc.

### <Berberrubine acetic acid adduct and method for producing the same>

In one embodiment, a berberrubine acetic acid adduct and a method for producing the same are provided.

A berberrubine acetic acid adduct can be produced by a manufacturing process that includes adding acetic acid to quinoid-type berberrubine. For example, 400 mL of acetic acid is added to quinoid-type berberrubine prepared from 50.0 g of berberine chloride, the mixture is stirred at 40 °C for 1 hour, then 500 mL of diethyl ether is added and the mixture is stirred for another 3 hours, then the mixture is subjected to suction filtration, and the filtrate is dried under reduced pressure at room temperature, thereby obtaining a berberrubine acetic acid adduct as orange-yellow crystals.

The berberrubine acetic acid adduct may be a berberrubine acetic acid adduct obtained by adding acetic acid and/or acetate ions to:
enol-type berberrubine represented by the following Formula (2): 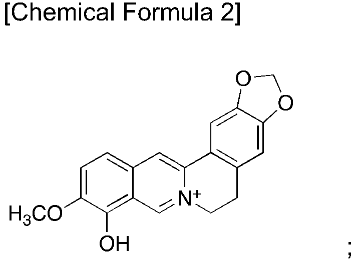 or zwitterionic-type berberrubine represented by the following Formula (3):

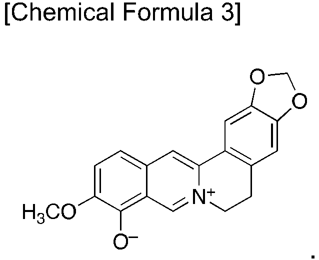

The berberrubine acetic acid adduct is a berberrubine acetic acid adduct in which 1 equivalent or more of acetic acid and/or acetate ions have been added to 1 equivalent of berberrubine. Moreover, in an embodiment, the berberrubine acetic acid adduct is a berberrubine acetic acid adduct in which more than 1 equivalent, more than 2 equivalents, more than 3 equivalents, more than 4 equivalents, more than 5 equivalents, more than 6 equivalents, or more than 7 equivalents of acetic acid and/or acetate ions are added to 1 equivalent of berberrubine, or a berberrubine acetic acid adduct in which 1 equivalent or more, 2 equivalents or more, 3 equivalents or more, 4 equivalents or more, 5 equivalents or more, 6 equivalents or more, or 7 equivalents or more of acetic acid and/or acetate ions are added to 1 equivalent of berberrubine. For example, the berberrubine acetic acid adduct may be 2 equivalents, 3 equivalents, 4 equivalents, 5 equivalents, 6 equivalents, 7 equivalents, 8 equivalents, or 9 equivalents, within a range of 1-10 equivalents or a range of any of these values combined as upper and lower limits, with respect to 1 equivalent of berberrubine. For example, the berberrubine acetic acid adduct is a berberrubine acetic acid adduct to which 2-10 equivalents, 2-9 equivalents, 3-8 equivalents, or 4-7 equivalents of acetic acid and/or acetate ions have been added.

The berberrubine acetic acid adduct in which the above range of equivalents of acetic acid and/or acetate ion have been added to 1 equivalent of berberrubine tends to have higher solubility in water.

The berberrubine acetic acid adduct may be a berberrubine acetic acid adduct that is:
(i) a compound represented by the following Formula (4): 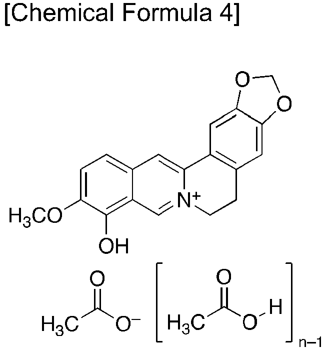 [wherein, n is 1 or more];
(ii) a compound represented by the following Formula (5): 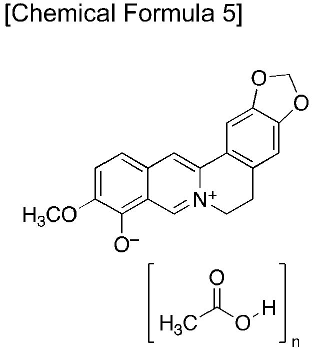 [wherein, n is 1 or more]; or
(iii) a mixture of the compound (i) and the compound (ii).

Here, n is not limited to an integer. In an embodiment, n is greater than 1, greater than 2, greater than 3, greater than 4, greater than 5, greater than 6, or greater than 7, or 1 or more, 2 or more, 3 or more, 4 or more, 5 or more, 6 or more, or 7 or more. Moreover, in an embodiment, n is in a range of 1-10, 2-9, 3-8, or 4-7. A berberrubine acetic acid adduct with n in these ranges tends to have higher solubility in water.

The structure of the berberrubine acetic acid adduct can be confirmed by the solid-state ¹³C NMR method. As the solid-state ¹³C NMR method, for example, the cross polarization magic-angle spinning (CPMAS) or dipole decoupling magic-angle spinning (DDMAS) methods can be used, and moreover, the equivalent amount of acetic acid and/or acetate ions added to 1 equivalent of berberrubine in the berberrubine acetic acid adduct and n in [Chemical Formula 4] and [Chemical Formula 5] can be determined by respectively calculating, with the solid-state ¹³C NMR analysis, the equivalent amount of berberrubine from the integral value of the peak of the 8th carbon of berberrubine at 26 or 27-28 ppm, and the equivalent amount of acetic acid and/or acetate ions from the integral value of the peak of the CH3 carbon atom of acetic acid at 20-25 ppm, and then dividing the equivalent amount of acetic acid and/or acetate ions by the equivalent amount of berberrubine. In addition, the equivalent amount of acetic acid and/or acetate ions added to 1 equivalent of berberrubine in the berberrubine acetic acid adduct and n in [Chemical Formula 4] and [Chemical Formula 5] can also be determined by respectively calculating the equivalent amount of berberrubine from the integral value of the two carbon peaks of berberrubine at 55 ppm and the equivalent amount of acetic acid and/or acetate ion from the integral value of the C=O carbon atom peak of acetic acid at 180 ppm, and then dividing the equivalent amount of acetic acid and/or acetate ions by the equivalent amount of berberrubine.

The solubility of the berberrubine acetic acid adduct in water may be 0.01 g/mL or more, 0.02 g/mL or more, 0.05 g/mL or more, 0.1 g/mL or more, 0.2 g/mL or more, 0.5 g/mL or more, or 1.0 g/mL or more. The solubility of the berberrubine acetic acid adduct in water can be adjusted by the amount of acetic acid and/or acetate ions added to berberrubine, as described above. The berberrubine acetic acid adduct in which 1 equivalent or more of acetic acid and/or acetate ions have been added to 1 equivalent of berberrubine or a berberrubine acetic acid adduct in which n in [Chemical Formula 4] and [Chemical Formula 5] is 1 or more tends to have higher solubility in water.

The solubility of the berberrubine acetic acid adduct (or other berberrubine-related compounds) in water or other solvents (e.g., methanol, dimethyl sulfoxide (DMSO), ethanol, or chloroform) can be determined by the following procedure, which is partially modified from the method described in the Japanese Pharmacopoeia General Rules. Add 1 g of a berberrubine-related compound to a solvent, stir the mixture for 30 seconds every 5 minutes at room temperature, and check whether the quinoid-type berberrubine and berberrubine-related compound have dissolved after 30 minutes. Determine the minimum amount (mL) of the solvent that can completely dissolve the berberrubine-related compound by changing the volume of the solvent, as appropriate. Calculate the solubility (g/mL) of each berberrubine-related compound in each solvent from the used berberrubine-related compound (1 g) and the minimum volume (mL) of the solvent.

A berberrubine acetic acid adduct having solubility in water in the above range tends to be easier to administer to or be ingested by living organisms, and easier to be blended into pharmaceutical, food, and agricultural compositions, etc.

### <Aqueous solution of berberrubine acetic acid adduct>

In one embodiment, an aqueous solution of a berberrubine acetic acid adduct is provided. The berberrubine acetic acid adduct releases the added acetic acid and/or acetate ions in the aqueous solution. Moreover, the berberrubine acetic acid adduct is typically an acetic acid adduct of enol-type berberrubine and/or zwitterionic-type berberrubine. Therefore, the aqueous solution of the berberrubine acetic acid adduct is an aqueous solution containing enol-type berberrubine and/or zwitterionic-type berberrubine.

The aqueous solution of the berberrubine acetic acid adduct may be an aqueous solution in which 0.0001 g or more, 0.001 g or more, 0.01 g or more, 0.05 g or more, or 0.1 g or more, or 0.0001-1.0 g, 0.001-1.0 g, 0.01-1.0 g, or 0.05-1.0 g of berberrubine acetic acid adduct is dissolved per 1 mL of water. This aqueous solution may contain 0.0001 g or more, 0.001 g or more, 0.01 g or more, 0.05 g or more, or 0.1 g or more, or 0.0001-1.0 g, 0.001-1.0 g, 0.01-1.0 g, or 0.05-1.0 g of enol-type berberrubine and/or zwitterionic-type berberrubine per 1 mL of water. Moreover, the enol-type berberrubine and/or zwitterionic-type berberrubine in this aqueous solution may have a molar concentration of 0.0002 M or more, 0.002 M or more, 0.02 M or more, 0.1 M or more, or 0.2 M or more, or 0.0002 M to 2 M, 0.002 M to 2 M, 0.02 M to 2 M, or 0.1 M to 2M.

The aqueous solution of the berberrubine acetic acid adduct may contain any other ingredients to the extent that they do not impair the effect of the present invention. Non-limiting examples of other ingredients include buffering agents, pH adjusters, surfactants, excipients, preservatives, antiseptics, medicinal ingredients, etc.

The aqueous solution in which a berberrubine acetic acid adduct is dissolved in these ranges is useful when administrated to or ingested by living organisms, and when blended in pharmaceutical, food, and agricultural compositions.

<Berberrubine acetic acid adduct and method for producing aqueous solution thereof>

In one embodiment, a berberrubine acetic acid adduct and a method for producing an aqueous solution of the berberrubine acetic acid adduct are provided.

The berberrubine acetic acid adduct can be preferably produced by a manufacturing process that includes adding acetic acid to quinoid-type berberrubine. Here, acetic acid may be added directly to a powder of quinoid-type berberrubine or may be added to a solution of quinoid-type berberrubine.

In an embodiment, acetic acid is added to quinoid-type berberrubine as a liquid. For example, 400 mL of acetic acid is added to 40 g of quinoid-type berberrubine, the mixture is stirred at 40 °C for 1 hour, then 500 mL of diethyl ether is added and the mixture is stirred for another 3 hours, then the mixture is subjected to suction filtration, and the filtrate is dried for 2 hours under reduced pressure (0.1 kPa) at room temperature, thereby making it possible to obtain a berberrubine acetic acid adduct as orange-yellow colored crystals. Here, the berberrubine acetic acid adduct is typically a berberrubine acetic acid adduct in which more than 1 equivalent of acetic acid and/or acetate ions have been added to 1 equivalent of berberrubine.

In another aspect, acetic acid is added to quinoid-type berberrubine as acetic acid vapor. For example, a berberrubine acetic acid adduct can be obtained as orange-yellow crystals by placing a beaker containing quinoid-type berberrubine in a sealed container saturated with acetic acid vapor for 1 week while stirring the mixture with a spatula every other day, and then subjecting the contents to washing with diethyl ether and suction filtration. The berberrubine acetic acid adduct is typically a berberrubine acetic acid adduct in which more than 1 equivalent of acetic acid and/or acetate ions have been added to 1 equivalent of berberrubine. Here, the amount of acetic acid and/or acetate ions added to berberrubine is the same as that in the berberrubine acetic acid adduct prepared by the method described above in which acetic acid is not added as acetic acid vapor.

A berberrubine acetate salt (a berberrubine acetic acid adduct in which 1 equivalent of acetic acid and/or acetate ions have been added to 1 equivalent of berberrubine) can be obtained as reddish-orange crystals by drying the berberrubine acetic acid adduct under reduced pressure at a temperature higher than room temperature (e.g., 80 °C) for 5 hours. In addition, the amount of acetic acid and/or acetate ions added to berberrubine can be adjusted by adjusting the temperature and time, as appropriate.

Conventionally, in order to add acetic acid and/or acetate ions to a compound, a method of adding acetic acid to a solution in which that compound is dissolved in an organic solvent (e.g., diethyl ether or tetrahydrofuran), and then precipitating the acetate adduct of the substance as an acetic salt that is insoluble in the organic solvent has been commonly used. This conventional method generally cannot create an acetic acid adduct of the compound to which more than 1 equivalent of acetic acid and/or acetate ions have been added. Meanwhile, unlike conventional methods, the method of the present embodiment does not require dissolving quinoid-type berberrubine in an organic solvent. In addition, it is possible to prepare a berberrubine acetic acid adduct to which more than 1 equivalent of acetic acid and/or acetate ions have been added. Furthermore, it is possible to adjust the amount of acetic acid and/or acetate ions added to berberrubine.

Dissolving the berberrubine acetic acid adduct (including berberrubine acetate salt) in water enables preparation of an aqueous solution of the berberrubine acetic acid adduct (including berberrubine acetate salt).

<Composition containing berberrubine acetic acid adduct or aqueous solution thereof>

In one embodiment, a composition containing a berberrubine acetic acid adduct or an aqueous solution thereof is provided.

The composition containing a berberrubine acetic acid adduct or an aqueous solution thereof may contain a berberrubine acetic acid adduct in any proportion to the extent that it does not impair the effect of the present invention. In an embodiment, the content of the berberrubine acetic acid adduct may be, with respect to the total composition containing the berberrubine acetic acid adduct, 0.00001 mass% or more, 0.0001 mass% or more, 0.001 mass% or more, 0.01 mass% or more, 0.1 mass% or more, 1 mass% or more, 2 mass% or more, 5 mass% or more, 10 mass% or more, 20 mass% or more, 30 mass% or more, 40 mass% or more, 50 mass% or more, 60 mass% or more, 70 mass% or more, 80 mass% or more, 90 mass% or more, 95 mass% or more, 98 mass% or more, 99 mass% or more, or 100 mass%. Moreover, the composition containing the berberrubine acetic acid adduct or the aqueous solution thereof may contain any other ingredients to the extent that they do not impair the effect of the present invention. Non-limiting examples of other ingredients include buffering agents, pH adjusters, surfactants, excipients, preservatives, antiseptics, medicinal ingredients, etc.

### <Application of composition>

In one embodiment, the composition containing quinoid-type berberrubine and the composition containing the berberrubine acetic acid adduct or the aqueous solution thereof may be used for the same application or for different applications. Moreover, the quinoid-type berberrubine or the composition containing the berberrubine acetic acid adduct or the aqueous solution thereof may be used for multiple applications or for a single application.

The quinoid-type berberrubine or the composition containing the berberrubine acetic acid adduct or the aqueous solution thereof may be administered to a subject in any form.

For example, the compositions of the present disclosure can be administered, for example, by oral administration, intravenous administration, intramuscular administration, intraspinal administration, sublingual administration, transrectal administration, ophthalmic administration, ear administration, otological administration, or transdermal administration, when the subject is a human or animals other than humans. However, when this composition is a food composition, the subject may ingest it orally. Moreover, when the subject is a plant, the composition can be administered to the plant by, for example, spraying or applying it to the plant, or adding it to soil, etc. as a fertilizer.

Moreover, the compositions of the present disclosure may also be administered to a subject in vitro. In such cases, the subject may be mitochondria, cells, or tissues. The mitochondria may be those isolated from cells, tissues, or individuals; the cells may be those isolated from tissues or individuals; and the tissues may be those isolated from individuals. The Cells may be primary cultured cells, subcultured cells, or cell lines. When administered to a subject in vitro, the compositions of the present disclosure can be administered to the subject by direct addition to the subject or by addition to the medium in which the subject is cultured.

### (Pharmaceutical composition)

In one embodiment, the quinoid-type berberrubine or the composition containing the berberrubine acetic acid adduct or the aqueous solution thereof may be a pharmaceutical composition.

A pharmaceutical composition includes, for example, compositions contained in prescription drugs, non-prescription drugs, or quasi-drugs. Moreover, a pharmaceutical composition may be intended for use in humans or in animals other than humans.

A pharmaceutical composition is preferably a composition for activating mitochondria, for treating or preventing mitochondrial diseases, for treating or preventing cancer, for treating or preventing muscle diseases, for treating or preventing neurological diseases, for treating or preventing cardiac diseases, for ameliorating or preventing cardiac hypertrophy, for treating infertility, for activating sperm activity, for preventing the formation of wrinkles in the skin, for reducing the depth and/or number of wrinkles, for inhibiting the increase in the depth and/or number of wrinkles, for inhibiting darkening of the skin, for ameliorating or preventing obesity, for slowing progression of aging, or for extending lifespan.

A pharmaceutical composition may be formulated with one or more other compositions, or may be formulated separately from one or more other compositions and used in combination.

"Treatment" includes the reduction, alleviation, or mitigation of symptoms of diseases, and "prevention" includes protection against the onset of a future disease or symptom and inhibition of its progression. Desired therapeutic effects of treatment include remission of symptoms, improvement of direct or indirect pathological consequences of disease, reduction in the rate of progression of symptom deterioration, recovery or alleviation of disease states, and improved prognosis. For example, treating or preventing cancer includes administering quinoid-type berberrubine or a pharmaceutical composition containing berberrubine acetic acid adduct or an aqueous solution thereof for the purpose of achieving at least one desired therapeutic effect, such as reducing the number of cancer cells, reducing tumor size, reducing the rate at which cancer cells invade peripheral organs, or reducing tumor metastasis or tumor growth, in a subject having cancer or having been diagnosed as having cancer.

A pharmaceutical composition may be, for example, tablets, granules, dispersions, capsules, emulsions, suspensions, syrups, or injections such as sterile solutions and suspension liquids. When two or more pharmaceutical compositions are separately formulated into two or more formulations, it is possible to administer the individual formulations simultaneously, or separately at regular time intervals, or consecutively. The two or more formulations may be administered respectively at different times per day. Moreover, a pharmaceutical composition may be administered systemically or topically, and orally or parenterally. When the active ingredients are separately formulated into two or more formulations, it is possible to administer the individual formulations via different paths.

### (Food composition)

In one embodiment, the quinoid-type berberrubine or the composition containing the berberrubine acetic acid adduct or the aqueous solution thereof may be a food composition.

A food composition includes, in addition to compositions contained in general food and beverage products, compositions, for example, contained in nutritional foods and nutritional beverages, luxury foods and luxury beverages, nutritional supplementary foods and beverages (supplements), and food additives. Moreover, a food composition may be intended for use in humans or in animals other than humans.

A food composition is preferably a composition for activating mitochondria, for ameliorating or preventing symptoms related to mitochondrial diseases, for ameliorating or preventing symptoms related to cancer, for ameliorating or preventing symptoms related to muscle diseases, for ameliorating or preventing symptoms related to neurological diseases, for ameliorating or preventing symptoms related to cardiac diseases, for ameliorating or preventing cardiac hypertrophy, for ameliorating symptoms related to infertility, for activating sperm activity, for preventing the formation of wrinkles in the skin, for reducing the depth and/or number of wrinkles, for inhibiting the increase in the depth and/or number of wrinkles, for inhibiting darkening of the skin, for ameliorating or preventing obesity, for slowing progression of aging, or for extending lifespan.

A food composition may be formulated with one or more other compositions, or may be formulated separately from one or more other compositions and used in combination.

A food composition may be in the form of beverages, solids, liquids, pastes, powders, etc. For example, a food composition may be beverages, soups, processed meat products, processed vegetable products, processed fruit products, paste-type seasonings, paste-type concentrated foods, granulated seasonings, granulated and concentrated foods, supplements, etc. Here, processed foods mean natural food materials that have been processed and/or cooked, and include frozen foods, retort pouch foods, canned foods, bottled foods, etc. Supplements (nutritional supplementary foods and beverages) may be, for example, in the form of liquid drinks, capsules, tablets, powders, etc. The food compositions of the present disclosure or foods, etc. containing the food compositions may be included in foods labeled as having the effects of activating mitochondria, ameliorating or preventing symptoms related to mitochondrial diseases, ameliorating or preventing symptoms related to cancer, ameliorating or preventing symptoms related to muscle diseases, ameliorating or preventing symptoms related to neurological diseases, ameliorating or preventing symptoms related to cardiac diseases, ameliorating or preventing cardiac hypertrophy, ameliorating symptoms related to infertility, activating sperm activity, preventing the formation of wrinkles in the skin, reducing the depth and/or number of wrinkles, inhibiting the increase in the depth and/or number of wrinkles, inhibiting darkening of the skin, ameliorating or preventing obesity, slowing progression of aging, or extending lifespan. The food composition or food may be provided in a sealed form in bags, containers, etc. The bags and containers used in the present disclosure may be any bags and containers normally used for food products.

### (Cosmetic composition)

In one embodiment, the quinoid-type berberrubine or the composition containing the berberrubine acetic acid adduct or the aqueous solution thereof may be a cosmetic composition.

A cosmetic composition includes, for example, compositions having the purpose of beauty treatment for the skin, hair, and nails. Moreover, a cosmetic composition may be intended for use in humans or in animals other than humans.

A cosmetic composition is preferably a composition for activating mitochondria, for preventing the formation of wrinkles in the skin, for reducing the depth and/or number of wrinkles, for inhibiting the increase in the depth and/or number of wrinkles, for inhibiting darkening of a skin, or for slowing progression of aging.

A cosmetic composition may be formulated with one or more other compositions, or may be formulated separately from one or more other compositions and used in combination.

A cosmetic composition is a composition having the purpose of beauty treatment for the skin, hair, nails, etc. and can be formulated in a form that allows care of these regions. For example, a cosmetic composition may be liquids, lotions, creams, patches, oils, sprays, liquid cleansers, solid soaps, etc. Moreover, the products thereof may be cosmetic liquids, serums, moisturizing lotions, moisturizing creams, soaps, body washes, bath salts, sunscreens, shaving lotions, depilatories, shampoos, conditioners, hair tonics, hair dyes, etc.

### (Agricultural composition)

In one embodiment, the quinoid-type berberrubine or the composition containing the berberrubine acetic acid adduct or the aqueous solution thereof may be an agricultural composition.

An agricultural composition includes, for example, a composition for activating mitochondria, a composition containing nutrition or nutritional supplementary ingredients for agricultural crops, etc., a composition for promoting or inhibiting growth of agricultural crops, etc., a composition used for promoting or inhibiting physiological functions of agricultural crops, etc., a composition for treating or preventing diseases of agricultural crops, etc. Agricultural crops, etc. include trees, bamboo, foliage plants, aquatic plants, and agricultural and forestry products.

An agricultural composition is preferably a composition for promoting growth of branches and leaves of plants of agricultural crops, etc., for promoting growth of flowers and fruits, for promoting root spreading, for slowing progression of aging, or for extending lifespan.

An agricultural composition may be formulated with one or more other compositions, or may be formulated separately from one or more other compositions and used in combination.

An agricultural composition can be formulated in solid form, such as powders, granules, encapsulated granules, fine granules, microgranule capsules, microcapsules, pellets, dispersible concentrates, tablets, flakes, or in liquid form with the active ingredients dissolved in liquid components. The products may be pesticides, fertilizers, potting soil with fertilizer, etc.

### (Composition for activating mitochondria)

In one embodiment, the quinoid-type berberrubine or the composition containing the berberrubine acetic acid adduct or the aqueous solution thereof may be a composition for activating mitochondria.

Activating mitochondria may be to improve the function of mitochondria (e.g., respiratory capacity), and moreover, to prevent the decline of the function of mitochondria.

Mitochondria are intracellular organelles of eukaryotes and have important functions in the normal life activity of eukaryotes, such as efficient production of ATP through aerobic respiration. The composition of the present disclosure can maintain individuals, tissues, and cells in a healthy state by effectively activating mitochondria, and can also prevent, improve, or treat mitochondrial diseases described below and various diseases and symptoms in which declining mitochondrial function is at least one of the causes of onset.

Moreover, the composition of the present disclosure can preferably be used as a pharmaceutical composition, food composition, cosmetic composition, or agricultural composition for activating mitochondria.

### (Compound for mitochondrial diseases)

In one embodiment, the quinoid-type berberrubine or the composition containing the berberrubine acetic acid adduct or the aqueous solution thereof may be a composition for treating or preventing mitochondrial diseases.

Mitochondrial diseases are diseases caused by abnormal mitochondrial function. Within this definition, mitochondrial diseases can include any form of disease, but non-limiting examples include neurological disease, muscle disease, cardiac disease, or infertility, discussed below, and neurological disease, muscle disease, or infertility in which declining mitochondrial function is at least one of the causes of onset. For example, mitochondrial diseases may be Parkinson's disease or mitochondrial encephalomyopathy, lactic acidosis, and stroke-like episode (MELAS). MELAS is a type of mitochondrial disease characterized by recurrent stroke-like attacks and is typically caused by a point mutation (3243A>G mutation) in mtDNA.

The composition of the present disclosure can preferably be used as a pharmaceutical composition or food composition for treating or preventing mitochondrial diseases.

### (Composition for neurological diseases)

In one embodiment, the quinoid-type berberrubine or the composition containing the berberrubine acetic acid adduct or the aqueous solution thereof may be a composition for treating or preventing neurological diseases.

Note that neurological diseases are diseases that cause neurological disorders due to lesions in the nerves. Within this definition, neurological disease can include any form of disease, but non-limiting examples include Parkinson's disease, dementia, Guillain-Barre syndrome, Pick's disease, and Huntington's disease. A non-limiting example of dementia is Alzheimer-type dementia. Neurological diseases may have declining mitochondrial function as at least one of the causes of onset, but are not limited thereto.

The treatment or prevention of neurological diseases may be the amelioration or prevention of at least one of the clinical symptoms recognized in said neurological diseases. The treatment or prevention of Parkinson's disease may be to ameliorate or prevent one or more of the symptoms recognized in Parkinson's disease, such as tremor (shaking), slow movement, muscle stiffness (muscle rigidity), and postural retention disorder (prone to falling). The treatment or prevention of dementia may to ameliorate or prevent one or more of the symptoms recognized in dementia, such as memory impairment, aphasia, executive dysfunction, etc. In an embodiment, the treatment or prevention of dementia may be inhibiting the pathological conditions seen in dementia, for example, the accumulation of tau proteins in brain neurons, or removing at least some of the tau proteins that have accumulated in the brain neurons.

Moreover, the treatment or prevention of neurological diseases may be inhibiting or at least partially eliminating the accumulation of tau proteins or inhibiting the phosphorylation of S396 and/or S422 of the tau proteins. Moreover, the treatment or prevention of neurological diseases may be inhibiting the activity of GSK3β or JNK.

The composition of the present disclosure can preferably be used as a pharmaceutical composition or food composition for treating or preventing neurological diseases.

### (Composition for muscle disease)

In one embodiment, the quinoid-type berberrubine or the composition containing the berberrubine acetic acid adduct or the aqueous solution thereof may be a composition for treating or preventing muscle diseases.

Muscular diseases are diseases resulting in disorder due to lesions in the muscles. Muscles include, for example, skeletal, visceral, smooth, and cardiac muscles. Within this definition, muscular diseases can include any form of disease, but non-limiting examples include Parkinson's disease, frailty, locomotive syndrome, sarcopenia, amyotrophic lateral sclerosis, myasthenia gravis, muscular dystrophy, polymyositis, cardiomyopathy (myocardial disease with cardiac dysfunction), mitochondrial encephalomyopathy, lactic acidosis, and stroke-like episode (MELAS). Muscular diseases may have declining mitochondrial function as at least one of the causes of onset, but are not limited thereto. Note that muscular diseases in the present disclosure include frailty, locomotive syndrome, and sarcopenia, and the present disclosure includes concepts having the purpose of promoting health and having the purpose of preventing the transition to more serious physical conditions.

Frailty is regarded as a state in which various organ functions change or reserve capacity declines along with aging, which increases vulnerability to external stress, whereas sarcopenia is a syndrome characterized by a progressive and systemic decrease in muscle mass and strength, which is associated with physical dysfunction, reduced quality of life, and risk of death. Locomotive syndrome is a state in which disorders of the motor system restrict daily life, resulting in a state that requires care and assistance, or a state of being at higher risk of requiring care and assistance. Sarcopenia is a concept included in locomotive syndrome, and sarcopenia and locomotive syndrome are concepts included in frailty. Mitochondrial dysfunction is recognized as one of the mechanisms for the development and progression of sarcopenia. The composition of the present disclosure can preferably be used as a pharmaceutical composition or food composition for treating or preventing frailty, locomotive syndrome, or sarcopenia.

### (Composition for cancer)

In one embodiment, the quinoid-type berberrubine or the composition containing the berberrubine acetic acid adduct or the aqueous solution thereof may be a composition for treating or preventing cancer.

Cancer refers to any cancer, including solid cancer and blood cancer, but non-limiting examples include epithelial carcinoma, carcinoma, cervical cancer, sarcoma, and myelodysplastic syndrome (MDS). Non-limiting examples of sarcoma include fibrosarcoma, odontogenic sarcoma, malignant fibrous histiocytoma, dermatofibrosarcoma, liposarcoma, rhabdomyosarcoma, leiomyosarcoma, angiosarcoma, Kaposi sarcoma, lymphangiosarcoma, synovial sarcoma, alveolar soft part sarcoma, extraskeletal chondrosarcoma, extraskeletal osteosarcoma, etc. Moreover, cancer may be inflammatory cancer. Inflammatory cancer may be cancer with inflammation or cancer caused by inflammation. Inflammation includes chronic and acute inflammation. Inflammatory cancer includes malignant cancers such as metastatic and/or invasive cancers. Inflammatory cancer may be, for example, cancers due to inflammation-associated carcinogenesis.

The composition of the present disclosure can preferably be used as a pharmaceutical composition or food composition for treating or preventing cancer.

### (Composition for cardiac disease and cardiac hypertrophy)

In one embodiment, the quinoid-type berberrubine or the composition containing the berberrubine acetic acid adduct or the aqueous solution thereof may be a composition for treating or preventing cardiac diseases and/or may be a compound for ameliorating or preventing cardiac hypertrophy.

Cardiac diseases include any disease or symptom related to the heart to the extent that the effect of the present invention is obtained and non-limiting examples thereof include cardiac failure, cardiomyopathy, valve disease, atrial septal defect, cardiac tumor, arrhythmia, cardiac hypertrophy, ischemic cardiac disease, etc. Note that cardiac failure is a state of various systemic ailments caused by decline in cardiac function, and its presence and progression can be effectively assessed by cardiac failure markers described below. Cardiomyopathy is a myocardial disease with cardiac dysfunction, and its presence and progression can be effectively assessed by myocardial fibrosis markers described below.

Cardiac hypertrophy is a state in which the myocardium and/or ventricular cavity is enlarged, which tends to reduce cardiac function and/or increase the risk of the cardiac diseases described above. Moreover, a cardiac disease may be a disease that includes cardiac hypertrophy as at least one of the symptoms and a disease that includes cardiac hypertrophy as at least one of the causes of onset.

The composition of the present disclosure may reduce the expression of cardiac failure markers in individuals, tissues, or cells. In this case, the composition of the present disclosure may reduce the expression of cardiac failure markers in individuals, tissues, or cells by preferably 5% or more or more preferably 10% or more, compared to the expression of the cardiac failure markers in individuals, tissues, or cells to which the composition of the present disclosure has not been administered.

As a cardiac failure marker, any cardiac failure marker may be used to the extent that the effect of the present invention can be obtained, and non-limiting examples thereof include one or more mRNAs of Nppa, Nppb, and Myh7.

As a myocardial fibrosis marker, any myocardial fibrosis marker may be used to the extent that the effect of the present invention can be obtained, and non-limiting examples thereof include one or more mRNAs of Collagen1 and Ctgf.

The composition of the present disclosure may reduce the expression of myocardial fibrosis markers in individuals, tissues, or cells. In this case, the composition of the present disclosure may reduce the expression of the myocardial fibrosis markers in individuals, tissues, or cells by preferably 5% or more or more preferably 10% or more, compared to the expression of the myocardial fibrosis markers in individuals, tissues, or cells to which the composition of the present disclosure has not been administered.

The composition of the present disclosure can preferably be used as a pharmaceutical composition or food composition for treating or preventing cardiac diseases.

### (Composition of infertility)

In one embodiment, the quinoid-type berberrubine or the composition containing the berberrubine acetic acid adduct or the aqueous solution thereof may be a compound for ameliorating infertility. The composition for ameliorating infertility may be a composition for ameliorating infertility in men, and the composition for ameliorating infertility in men may be a composition for activating sperm activity. Infertility may have declining mitochondrial function as at least one of the causes of onset, but are not limited thereto.

The composition of the present disclosure can preferably be used as a pharmaceutical composition or food composition for ameliorating infertility.

### (Composition for beauty treatment)

In one embodiment, the quinoid-type berberrubine or the composition containing the berberrubine acetic acid adduct or the aqueous solution thereof may be a composition having the purpose of beauty treatment for the regions of the skin, hair, nails, etc., and may preferably be a composition for ameliorating skin conditions or inhibiting or preventing deterioration of skin conditions.

Skin conditions include wrinkles, darkening (blotches), warts, sagging, dryness, etc. Deterioration of skin conditions includes, for example: for wrinkles, the formation of wrinkles or an increase in the depth and/or number of wrinkles; for darkening, occurrence of darkening or intensification of the darkened area and/or enlargement of the darkened area; for warts, the occurrence of warts or an increase in the size and/or number of warts; for sagging, the occurrence of sagging or an increase in the size and/or number of sagging; and for drying, the occurrence of dryness and/or an enlargement of the dry areas.

For example, the quinoid-type berberrubine or the composition containing the berberrubine acetic acid adduct or the aqueous solution thereof may be a composition for preventing the formation of wrinkles in the skin, a composition for reducing the depth and/or number of wrinkles, and/or a composition for inhibiting an increase in the depth and/or number of wrinkles, or a composition for inhibiting darkening of the skin (occurrence of darkening or intensification of the darkened area and/or enlargement of the darkened area).

The target skin is the skin of humans or animals other than humans, preferably that of a mammal, which is a human. Regions of the skin in a subject include the hands, face (forehead, cheeks, eye contour (around the eyeballs), corners of the eyes, under the eyes, eyelids, etc.), neck, legs, feet, arms, elbows, abdomen, back, etc. Wrinkles or darkening is not limited by its cause and may be caused by UV irradiation, aging, or side effects of pharmaceuticals.

The composition of the present disclosure can preferably be used as a pharmaceutical composition, food composition, or cosmetic composition for the purpose of beauty treatment.

### (Composition for ameliorating or preventing obesity)

In one embodiment, the quinoid-type berberrubine or the composition containing the berberrubine acetic acid adduct or the aqueous solution thereof may be a composition for ameliorating or preventing obesity. The composition for treating or preventing obesity may be a composition for reducing or preventing the accumulation of body fat. Body fat may be visceral fat or subcutaneous fat. That is, obesity may be visceral fat obesity or subcutaneous fat obesity. Reduction in accumulation of body fat may be due, for example, to reduction in white fat cells. The reduction of white fat cells is preferably due to the activation of brown fat cells. The activation of brown fat cells is preferably the activation of mitochondrial function in the brown fat cells.

The composition of the present disclosure can preferably be used as a pharmaceutical composition or food composition for treating or preventing obesity.

### (Composition for slowing aging)

In one embodiment, the quinoid-type berberrubine or the composition containing the berberrubine acetic acid adduct or the aqueous solution thereof may be a composition for slowing progression of aging.

Aging refers to the progression of changes in physical characteristics, decline in the function of tissues and cells, etc. associated with aging. Non-limiting examples of aging include increased risk of disease associated with aging, decline in muscle strength, skin aging (increased depth and/or number of wrinkles, increased blotches, warts, and sagging, etc.), and hair aging (graying, volume decrease, hair quality decrease). Non-limiting examples of diseases for which the risk of disease increases with aging include mitochondrial disease, cancer, muscle disease, neurological disease, cardiac disease, etc.

The composition of the present disclosure can preferably be used as a pharmaceutical composition, food composition, or cosmetic composition for slowing progression of aging.

### (Composition for extending lifespan)

In one embodiment, the quinoid-type berberrubine or the composition containing the berberrubine acetic acid adduct or the aqueous solution thereof may be a composition for extending lifespan or survival time. Here, lifespan may be the overall survival time of an individual, tissue, and/or cell from birth to death that is not due to external causes of death (e.g., traffic accidents). Survival time may be the overall survival time of a tissue and/or cell from birth to death, and may be the survival time from the start of administration of the quinoid-type berberrubine or the composition containing the berberrubine acetic acid adduct or the aqueous solution thereof until death.

The composition of the present disclosure can extend the lifespan or survival time of an individual, tissue, and/or cell by preferably 5 weeks or more, more preferably 10 weeks or more, and especially preferably 20 weeks or more, compared to the lifespan or survival time of an individual, tissue, and/or cell to which the composition of the present disclosure was not administered. Moreover, the composition of the present disclosure can extend the lifespan or survival time of a human by preferably 150 weeks or more, more preferably 300 weeks or more, and especially preferably 600 weeks or more, compared to the lifespan or survival time of a human to which the composition of the present disclosure was not administered. In addition, the composition of the present disclosure can extend the lifespan or survival time of an individual, tissue, and/or cell to preferably 1.05 times or more, more preferably 1.1 times or more, and especially preferably 1.2 times or more, compared to the lifespan or survival time of an individual, tissue, and/or cell to which the composition of the present disclosure was not administered.

Furthermore, the composition of the present disclosure can extend the 50% survival time of an individual, tissue, and/or cell by preferably 5 weeks or more, more preferably 10 weeks or more, and especially preferably 20 weeks or more, compared to the 50% survival time of an individual, tissue, and/or cell to which the composition of the present disclosure was not administered. Moreover, the composition of the present disclosure can extend the 50% survival time of a human by preferably 150 weeks or more, more preferably 300 weeks or more, and especially preferably 600 weeks or more, compared to the 50% survival time of a human to which the composition of the present disclosure was not administered. In addition, the composition of the present disclosure can extend the 50% survival time of an individual, tissue, and/or cell by preferably 1.05 times or more, more preferably 1.1 times or more, and especially preferably 1.2 times or more, compared to the 50% survival time of an individual, tissue, and/or cell to which the composition of the present disclosure was not administered. Note that 50% survival time may be a 50% survival time from the birth of an individual, tissue, and/or cell, or a 50% survival time from the start of administration of the composition of the present disclosure.

The composition of the present disclosure can preferably be used as a pharmaceutical composition or food composition for extending lifespan.

### (Composition for promoting plant growth)

In one embodiment, the quinoid-type berberrubine or the composition containing the berberrubine acetic acid adduct or the aqueous solution thereof may be a composition for promoting the growth of plants.

The target plant may be either a seed plant or a non-seed plant, but is preferably a seed plant. A seed plant may be either an angiosperm or gymnosperm, and a gymnosperm may be a plant classified into any of Cycadaceae, Ginkgoceae, Pinaceae, Taxaceae, Gnetocephala, etc. An angiosperm may be any plant classified into Monocotyledoneae, Magnoliopsida, etc. Plant growth promotion may have one or more effects related to promoting the growth of branches and leaves of plants of agricultural crops, etc., promoting the growth of flowers and fruits, and/or promoting root spreading.

The composition of the present disclosure can preferably be used as an agricultural composition for promoting the growth of plants.

Further aspects in the present disclosure are illustrated below.
[1] A composition containing quinoid-type berberrubine represented by the following Formula (I) 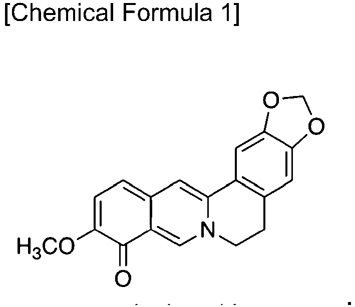
[2] The composition according to [1], wherein the compound represented by Formula (I) is isolated quinoid-type berberrubine.
[3] The composition according to [1] or [2], wherein the composition is a pharmaceutical composition.
[4] The composition according to any of [1] to [3], wherein the composition is a food composition.
[5] The composition according to any of [1] to [4], wherein the composition is a cosmetic composition.
[6] The composition according to any of [1] to [5], wherein the composition is an agricultural composition.
[7] The composition according to any of [1] to [6], wherein the composition is for activating mitochondria.
[8] The composition according to any of [1] to [7], wherein the composition is for treating or preventing mitochondrial diseases.
[9] The composition according to any of [1] to [8], wherein the composition is for treating or preventing Parkinson's disease.
[10] The composition according to any of [1] to [9], wherein the composition is for treating or preventing mitochondrial encephalomyopathy, lactic acidosis, and stroke-like episodes (MELAS).
[11] The composition according to any of [1] to [10], wherein the composition is for treating or preventing a cancer.
[12] The composition according to [11], wherein the cancer is epithelial carcinoma, carcinoma, cervical cancer, sarcoma, or myelodysplastic syndrome (MDS).
[13] The composition according to any of [1] to [12], wherein the composition is for treating or preventing a muscle disease.
[14] The composition according to [13], wherein the muscle diseases are frailty, locomotive syndrome, or sarcopenia.
[15] The composition according to any of [1] to [14], wherein the composition is for treating or preventing a neurological disease.
[16] The composition according to [15], wherein the neurological disease is dementia.
[17] The composition according to any of [1] to [16], wherein the composition is for treating or preventing cardiac diseases.
[18] The composition according to [17], wherein the cardiac disease is cardiac failure.
[19] The composition according to [17] or [18], wherein the cardiac disease is cardiomyopathy.
[20] The composition according to any of [1] to [20], wherein the composition is for ameliorating or preventing cardiac hypertrophy.
[21] The composition according to any of [1] to [20], wherein the composition is for ameliorating infertility.
[22] The composition according to any of [1] to [21], wherein the composition is for activating sperm activity.
[23] The composition according to any of [1] to [22], wherein the composition is for preventing formation of wrinkles in the skin, for reducing the depth and/or number of wrinkles, and/or for inhibiting an increase in the depth and/or number of wrinkles.
[24] The composition according to any of [1] to [23], wherein the composition is for inhibiting or preventing darkening of the skin.
[25] The composition according to any of [1] to [24], wherein the composition is for ameliorating or preventing obesity.
[26] The composition according to any of [1] to [25], wherein the composition is for slowing progression of aging.
[27] The composition according to any of [1] to [26], wherein the composition is for extending lifespan.
[28] The composition according to any of [1] to [27], wherein the composition is for promoting plant growth.
[29] A production method of a berberrubine acetic acid adduct, the method including:
   adding acetic acid to the quinoid-type berberrubine represented by the following Formula (I) 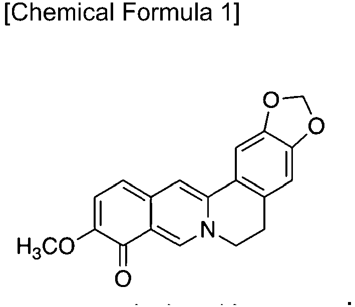
[30] The production method according to [29], comprising adding acetic acid as acetic acid vapor.
[31] A production method of an aqueous solution of a berberrubine acetic acid adduct, the method comprising:
   (i) adding acetic acid to the quinoid-type berberrubine represented by the following Formula (I) to produce a berberrubine acetic acid adduct 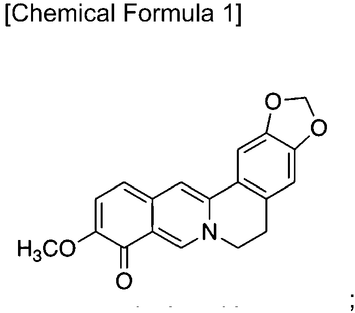 and
   (ii) dissolving the produced berberrubine acetic acid adduct in water.
[32] The production method according to [31], wherein in (i), acetic acid is added as acetic acid vapor to produce a berberrubine acetic acid adduct.
[33] A berberrubine acetic acid adduct.
[34] The berberrubine acetic acid adduct according to [33], wherein acetic acid or acetic acid ions are added to:
   enol-type berberrubine represented by the following Formula (2): 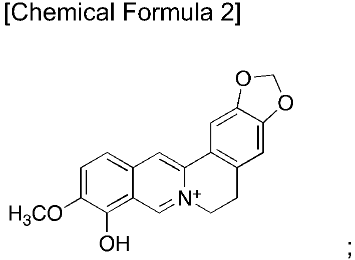 or zwitterionic-type berberrubine represented by the following Formula (3): 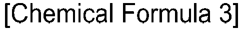 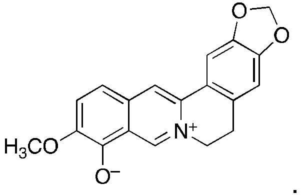
[35] The berberrubine acetic acid adduct according to [33] or [34], wherein 1 equivalent or more of acetic acid and/or acetate ions are added to 1 equivalent of berberrubine.
[36] The berberrubine acetic acid adduct according to any one of [33] to [35], wherein the berberrubine acetic acid adduct is:
   (i) a compound represented by the following Formula (4): 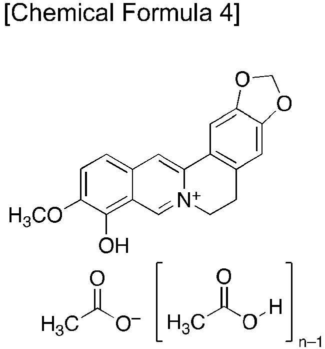 [wherein, n is one or more];
   (ii) a compound represented by the following Formula (5): 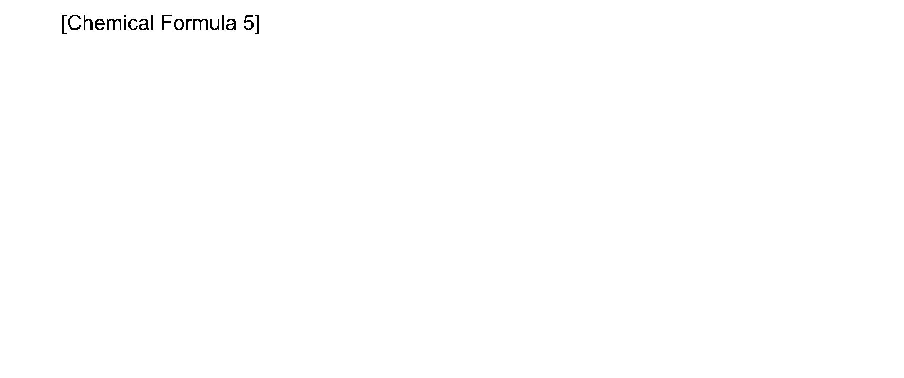 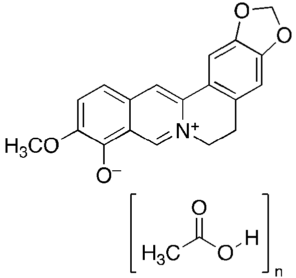 [wherein, n is one or more]; or
   (iii) a mixture of the compound (i) and the compound (ii).
[37] The berberrubine acetic acid adduct according to [33] to [36], wherein the solubility in is 0.01 g/mL or more.
[38] An aqueous solution of the berberrubine acetic acid adduct according to any of [33] to [37].
[39] A composition comprising the berberrubine acetic acid adduct according to any of [33] to [37] or the aqueous solution according to [38].
[40] The composition according to [39], wherein the composition is a pharmaceutical composition.
[41] The composition according to [39] or [40], wherein the composition is a food composition.
[42] The composition according to any of [39] to [41], wherein the composition is a cosmetic composition.
[43] The composition according to any of [39] to [42], wherein the composition is an agricultural composition.
[44] The composition according to any of [39] to [43], wherein the composition is for activating mitochondria.
[45] The composition according to any of [39] to [44], wherein the composition is for treating or preventing mitochondrial diseases.
[46] The composition according to any of [39] to [42], wherein the composition is for treating or preventing Parkinson's disease.
[47] The composition according to any of [39] to [46], wherein the composition is for treating or preventing mitochondrial encephalomyopathy, lactic acidosis, and stroke-like episode (MELAS).
[48] The composition according to any of [39] to [47], wherein the composition is for treating or preventing cancer.
[49] The composition according to [48], wherein the cancer is epithelial carcinoma, carcinoma, cervical cancer, sarcoma, or myelodysplastic syndrome (MDS).
[50] The composition according to any of [39] to [49], wherein the composition is for treating or preventing muscle diseases.
[51] The composition according to [50], wherein the muscle diseases are frailty, locomotive syndrome, or sarcopenia.
[52] The composition according to any of [39] to [51], wherein the composition is for treating or preventing neurological diseases.
[53] The composition according to [52], wherein the neurological disease is dementia.
[54] The composition according to any of [39] to [53], wherein the composition is for treating or preventing cardiac diseases.
[55] The composition according to [54], wherein the cardiac disease is cardiac failure.
[56] The composition according to [54] or [55], wherein the cardiac disease is cardiomyopathy.
[57] The composition according to any of [39] to [56], wherein the composition is for ameliorating or preventing cardiac hypertrophy.
[58] The composition according to any of [39] to [57], wherein the composition is for ameliorating infertility.
[59] The composition according to any of [39] to [58], wherein the composition is for activating sperm activity.
[60] The composition according to any of [39] to [59], wherein the composition is for preventing formation of wrinkles in the skin, for reducing the depth and/or number of wrinkles, and/or for inhibiting an increase in the depth and/or number of wrinkles.
[61] The composition according to any of [39] to [60], wherein the composition is for inhibiting or preventing darkening of the skin.
[62] The composition according to any of [39] to [61], wherein the composition is for ameliorating or preventing obesity.
[63] The composition according to any of [39] to [62], wherein the composition is for slowing progression of aging.
[64] The composition according to any of [39] to [63], wherein the composition is for extending lifespan.
[65] The composition according to any of [39] to [64], wherein the composition is for promoting plant growth.
[66] A method for treating a disease in a subject in need of the composition according to any of [1] to [28] and [39] to [65], the method including administering the composition to the subject.
[67] A method for ameliorating a symptom in a subject in need of the composition according to any of [1] to [28] and [39] to [65], the method including administering the composition to the subject.
[68] A use in a preparation of a pharmaceutical composition of quinoid-type berberrubine represented by the following Formula (I) 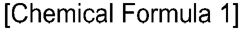 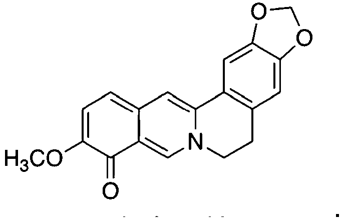
[69] A use in a preparation of a food composition of quinoid-type berberrubine represented by the following Formula (I) 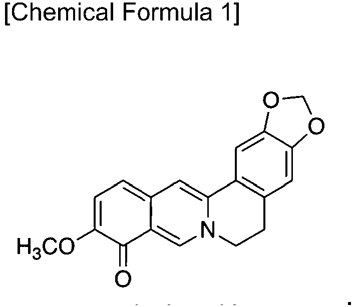
[70] A use in a preparation of a cosmetic composition of quinoid-type berberrubine represented by the following Formula (I) 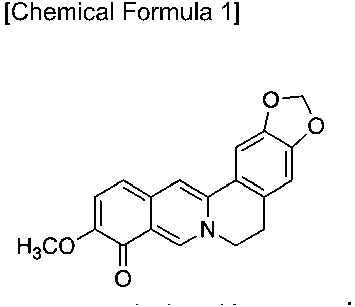
[71] A use in a preparation of an agricultural composition of quinoid-type berberrubine represented by the following Formula (I) 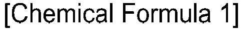 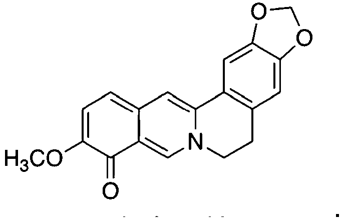
[72] Quinoid-type berberrubine represented by the following Formula (I): 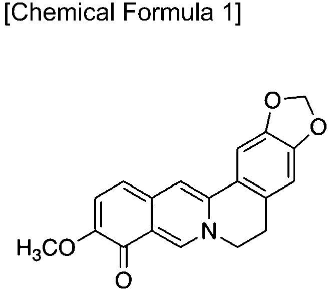 for use in treating or preventing diseases in a subject in need of the quinoid-type berberrubine.
[73] Quinoid-type berberrubine represented by the following Formula (I): 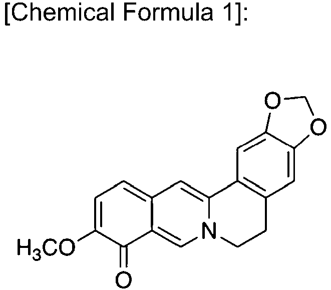 for use in ameliorating or preventing symptoms in a subject in need of the quinoid-type berberrubine.
[74] Quinoid-type berberrubine represented by the following Formula (I): 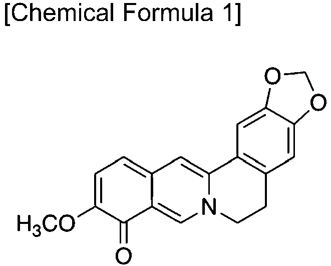 for use for the purpose of beauty treatment for a subject in need of the quinoid-type berberrubine.
[75] Quinoid-type berberrubine represented by the following Formula (I): 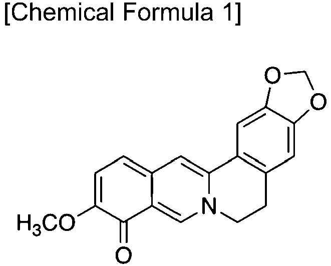 for use for an agricultural purpose in a subject in need of the quinoid-type berberrubine.
[76] Use of the berberrubine acetic acid adduct according to any of [33] to [37] or the aqueous solution according to [38] in the preparation of a pharmaceutical composition.
[77] Use of the berberrubine acetic acid adduct according to any of [33] to [37] or the aqueous solution according to [38] in the preparation of a cosmetic composition.
[78] Use of the berberrubine acetic acid adduct according to any of [33] to [37] or the aqueous solution according to [38] in the preparation of an agricultural composition.
[79] The berberrubine acetic acid adduct according to any of [33] to [37] or the aqueous solution according to [38] for use in treating a disease in a subject in need thereof.
[80] The berberrubine acetic acid adduct according to any of [33] to [37] or the aqueous solution according to [38] for use in ameliorating symptoms in a subject in need thereof.
[81] The berberrubine acetic acid adduct according to any of [33] to [37] or the aqueous solution according to [38] for the purpose of beauty treatment for a subject in need thereof.
[82] The berberrubine acetic acid adduct according to any of [33] to [37] or the aqueous solution according to [38] for an agricultural purpose in a subject in need thereof.

### EXAMPLES

Hereinafter, the present invention shall be explained using examples, but these examples do not limit the present invention. Note that unless otherwise indicated, commercially available reagents, devices, etc. described in the examples were used in accordance with the instructions or established methods of the manufacturer.

### [EXAMPLE 1]

### PREPARATION OF BERBERRUBINE-RELATED COMPOUND

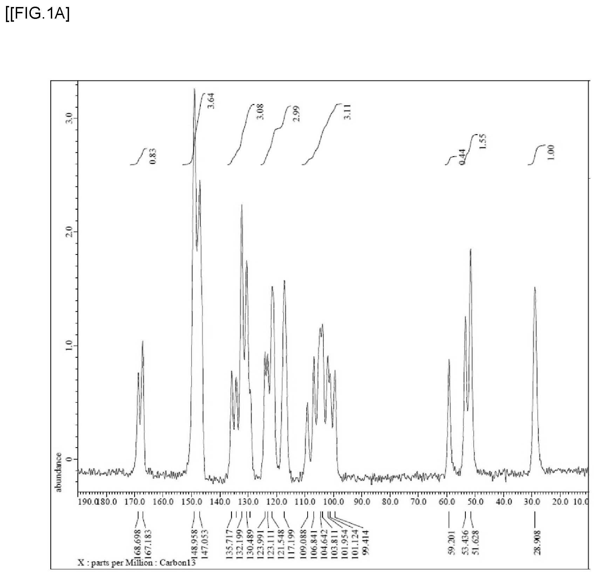

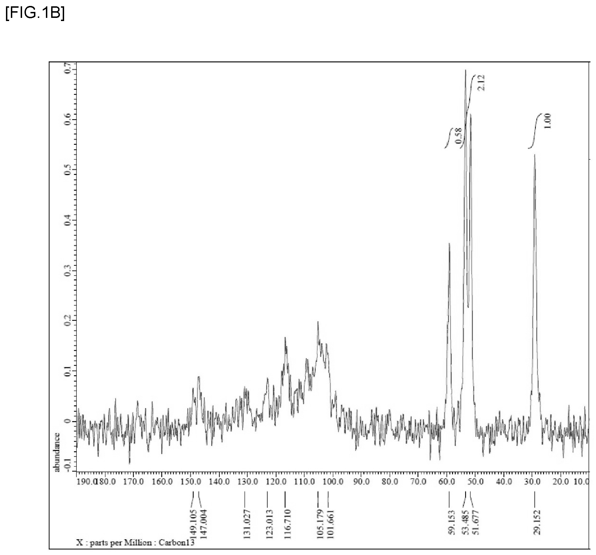

Quinoid-type berberrubine was prepared by heating commercially available berberine chloride (manufactured by NACALAI TESQUE, INC.) at 190 °C under reduced pressure for 3 hours. The solid-state ¹³C NMR using CPMAS and DDMAS methods, shown in FIGs. 1A and 1B, confirmed that the prepared quinoid-type berberrubine has an appropriate structure.

400 mL of acetic acid was added to the quinoid-type berberrubine prepared from 50.0 g of berberine chloride. Next, the mixture was stirred at room temperature for 1 hour, then 500 mL of diethyl ether was added. After stirring for another 3 hours, the filtrate was collected by suction filtration. The filtrate was dried at room temperature under reduced pressure for 2 hours, thereby obtaining a berberrubine acetic acid adduct as orange-yellow crystals.

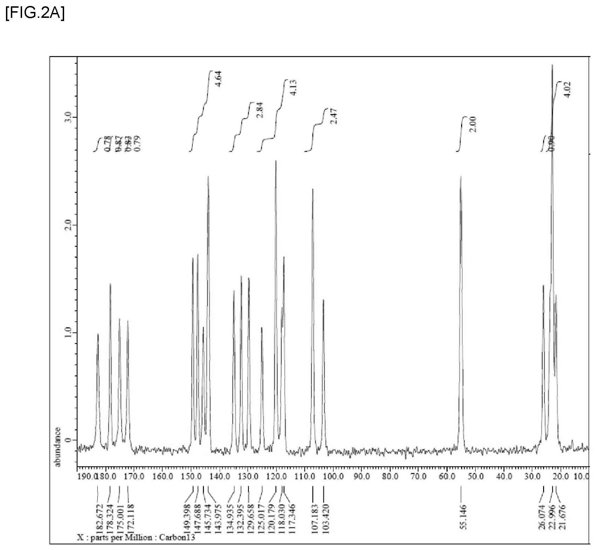

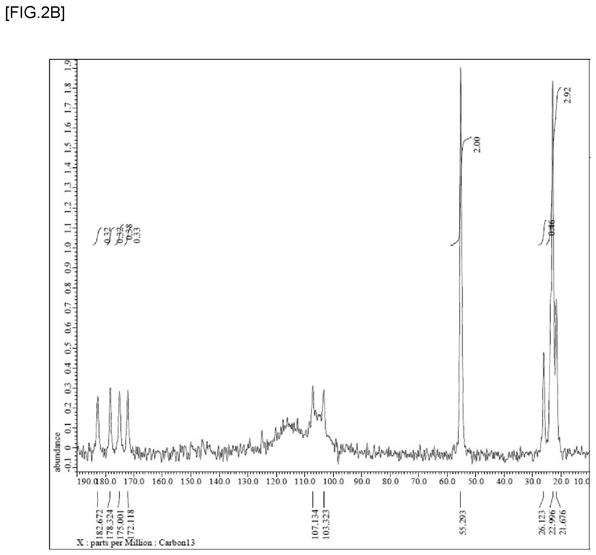

Using the solid-state ¹³C NMR using CPMAS and DDMAS methods, respectively shown in FIGs. 2A and 2B, the berberrubine acetic acid adduct was determined to be:
(i) a compound represented by the following Formula (4): 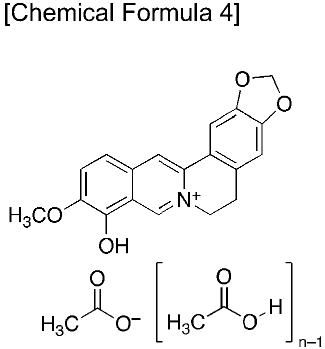 [wherein, n is 1 or more];
(ii) a compound represented by the following Formula (5): 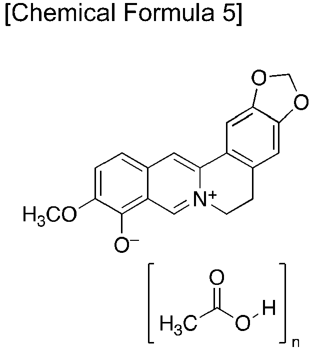 [wherein, n is 1 or more]; or
(iii) a mixture of the compound (i) and the compound (ii).

That is, the berberrubine acetic acid adduct prepared in Example 1 was the acetic acid adduct of enol-type berberrubine and/or zwitterionic-type berberrubine.

Here, n was determined by respectively calculating, in the solid-state ¹³C NMR, the equivalent amount of berberrubine from the integral value of the peak of the 8th carbon of berberrubine at 26 or 27-28 ppm, and the equivalent amount of acetic acid and/or acetate ions from the integral value of the peak of the CH3 carbon atom of acetic acid at 20-25 ppm, and then dividing the equivalent amount of acetic acid and/or acetate ions by the equivalent amount of berberrubine.

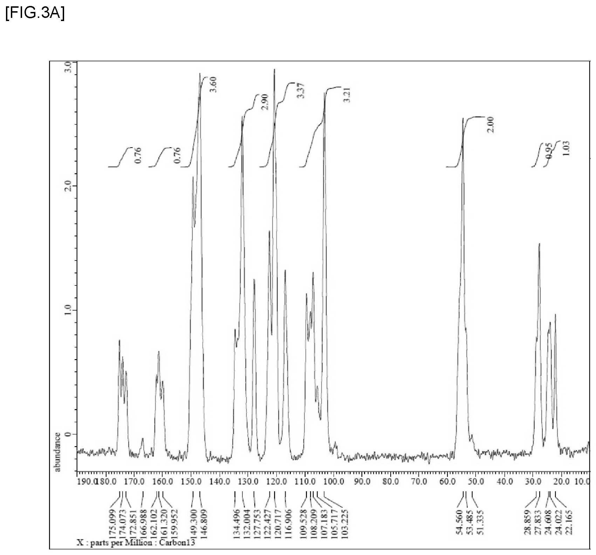

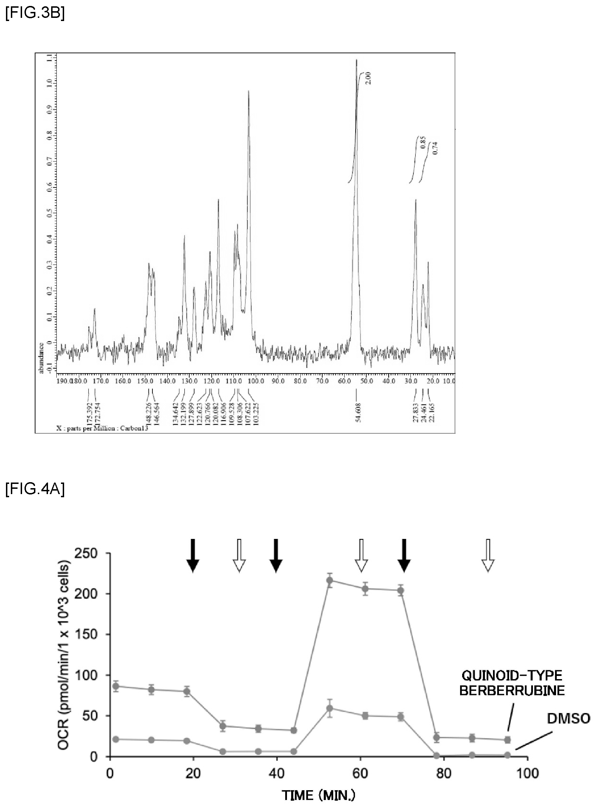

Moreover, the berberrubine acetic acid adduct was further dried under reduced pressure at 80 °C for 5 hours, thereby obtaining a berberrubine acetate salt (a berberrubine acetic acid adduct in which n=1 in Formulas (4) and (5) above) as red-orange crystals. The structure of the berberrubine acetate salt was estimated by the solid-state¹³ C NMR using CPMAS and DDMAS methods, shown in FIGs. 3A and 3B, and n was calculated as described above.

The experimental values of n determined for the berberrubine acetic acid adduct and berberrubine acetate salt described above are shown in Table 1.

**Table 1**

| | n (EXPERIMENTAL VALUES) | |
|---|---|---|
| | CPMAS METHOD | DDMAS METHOD |
| BERBERRUBINE ACETIC ACID ADDUCT | 4.5 | 6.3 |
| BERBERRUBINE ACETATE SALT | 1.1 | 0.9 |

### [EXAMPLE 2]

### SOLUBILITY OF BERBERRUBINE-RELATED COMPOUND

The solubility of quinoid-type berberrubine, a berberrubine acetic acid adduct, and berberrubine acetate salt was measured with respect to water, methanol, dimethyl sulfoxide (DMSO), ethanol, and chloroform.

Measurements were performed by the following procedure, which is partially modified from the method described in the Japanese Pharmacopoeia General Rules. A solvent (water, methanol, dimethyl sulfoxide (DMSO), ethanol, or chloroform) was added to 1 g of berberrubine-related compound (quinoid-type berberrubine, a berberrubine acetic acid adduct, or berberrubine acetate salt) and stirred for 30 seconds every 5 minutes at room temperature, and after 30 minutes, it was confirmed whether each berberrubine-related compound had dissolved. By changing the volume of the solvent, as appropriate, the minimum amount (mL) of the solvent that can completely dissolve the berberrubine-related compound was determined. From the used berberrubine-related compound (1 g) and the minimum volume (mL) of the solvent, the solubility (g/mL) of each berberrubine-related compound in each solvent was determined.

Results are shown in Table 2.

**Table 2**

| | SOLUBILITY (g/mL) OF BERBERRUBINE-RELATED COMPOUND IN 1 mL OF SOLVENT | | | | |
|---|---|---|---|---|---|
| SOLVENT | WATER | METHANOL | DMSO | ETHANOL | CHLOROFORM |
| BERBERRU BINE ACETIC ACID ADDUCT | 1.1 | 1.1 | 1.1 | 0.11 | 0.11 |
| BERBERRU BINE ACETIC ACETATE SALT | 0.030 | 0.034 | - | - | - |
| QUINOID-TYPE BERBERRU BINE | 0.000013 (LITERAT URE VALUE) | - | - | - | - |

As shown in Table 2, the berberrubine acetic acid adduct was extremely soluble in water. The solubility of the berberrubine acetic acid adduct in water was about 36 times greater than that of berberrubine acetate salt in water, and about 2300 times greater than that of quinoid-type berberrubine in water.

High water solubility of a drug is important in administering the drug to or allowing ingestion by living organisms, or when blending with compositions containing drugs, such as pharmaceutical compositions, food compositions, cosmetic compositions, and agricultural compositions. Therefore, the berberrubine acetic acid adduct of the present disclosure having high water solubility is useful for these applications.

### [EXAMPLE 3]

### EFFECTS ON BASAL RESPIRATION, ATP SYNTHESIS, AND MAXIMAL RESPIRATION IN MITOCHONDRIA

A DMSO solution of 3.5 mg/mL of quinoid-type berberrubine was added to C2C12 cells in Dulbecco's modified Eagle (DMEM) medium containing 10% fetal bovine serum (FBS), penicillin, and streptomycin, such that the final concentration was 30 µM (FIG. 4A).

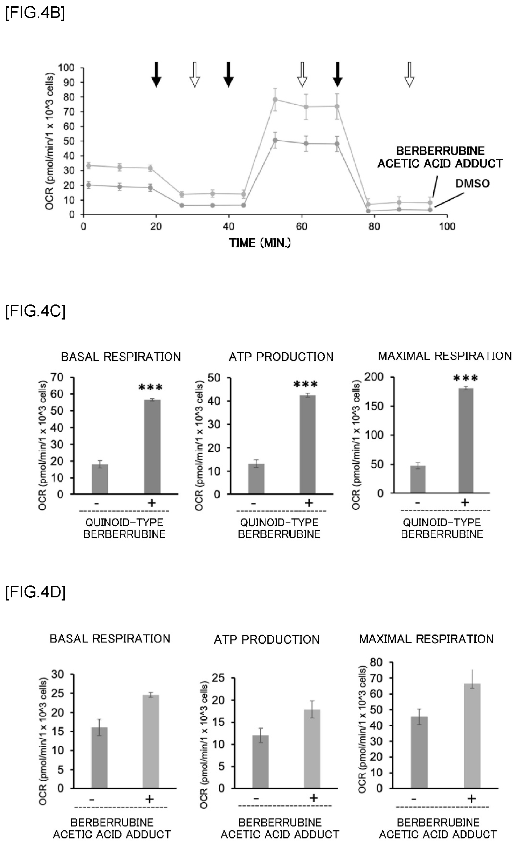

Moreover, 3.5 mg/mL of aqueous solution of a berberrubine acetic acid adduct was added to the C2C12 cells described above, such that the final concentration was 30 µM (FIG. 4B). As a control, the same amount of water was added to the C2C12 cells described above.

The point at which 24 hours had passed after addition of the berberrubine acetic acid adduct was set to 0 hours (minutes), and at the point of 20 minutes, an ATP synthase inhibitor (oligomycin; final concentration: 10 µM) was additionally added, at the point of 40 minutes, an uncoupling agent (carbonylcyanide-m-chlorophenylhydrazone (CCCP); final concentration: 10 µM) was additionally added, and at the point of 70 minutes, mitochondrial complex inhibitors (rotenone and antimycin; final concentration: 10 µM) was additionally added. During this period, the oxygen consumption rate (OCR) of the C2C12 cells was measured over time using an extracellular flux analyzer.

The OCR during the period between the addition of the ATP synthase inhibitor and the addition of the uncoupling agent, the OCR during the period between the addition of the uncoupling agent and the addition of the mitochondrial complex inhibitor, and the OCR in the period after the addition of the mitochondrial complex inhibitor each correlate with the amount of basal respiration, ATP synthesis, and maximal respiration in mitochondria. Thus, the amount of basal respiration, ATP synthesis, and maximal respiration in mitochondria were evaluated using OCR at the points of 30, 60, and 90 minutes as indicators.

As shown in FIG. 4C, the addition of quinoid-type berberrubine significantly enhanced the basal respiration, ATP synthesis, and maximal respiration in mitochondria by about 4-fold, compared to the controls.

Moreover, as shown in FIG. 4D, the addition of the berberrubine acetic acid adduct significantly enhanced the basal respiration, ATP synthesis, and maximal respiration in mitochondria by about 1.6-fold, respectively, compared to the controls.

These results indicate that the addition of the quinoid-type berberrubine and the addition of the berberrubine acetic acid adduct both improve mitochondrial function. Therefore, the quinoid-type berberrubine and berberrubine acetic acid adduct can provide an advantageous effect on diseases and symptoms caused by mitochondrial disorders.

Furthermore, the above results show that the addition of the quinoid-type berberrubine enhances the ATP synthesis by about 2.5-fold, the maximal respiration by about 2.6-fold, and the basal respiration by about 2.5-fold in mitochondria, compared to the addition of the berberrubine acetic acid adduct.

Therefore, the quinoid-type berberrubine can provide a more advantageous effect on diseases and symptoms caused by mitochondrial disorders compared to the berberrubine acetic acid adduct.

Moreover, the berberrubine acetic acid adduct used herein is an acetic acid adduct of enol-type berberrubine and/or zwitterionic-type berberrubine, as described above. Moreover, the acetic acid (or acetate ions) contained in the berberrubine acetic acid adduct is released in a sample (in water), so the berberrubine acetic acid adduct acts as enol-type berberrubine and/or zwitterionic-type berberrubine. Therefore, compared to the enol-type berberrubine and/or zwitterionic-type berberrubine, the quinoid-type berberrubine can provide a more advantageous effect on diseases and symptoms caused by mitochondrial disorders.

### [EXAMPLE 4]

### EFFECTS ON FORMATION OF MITOCHONDRIAL RESPIRATORY CHAIN SUPERCOMPLEXES

C2C12 cells transfected with green fluorescent protein (GFP)-bound complex I and red fluorescent protein (RFP)-bound complex IV were cultured in Dulbecco's modified Eagle (DMEM) medium containing 10% fetal bovine serum (FBS), penicillin, and streptomycin, and 3.5 mg/mL of a DMSO solution of quinoid-type berberrubine or 3.5 mg/mL of an aqueous solution of a berberrubine acetic acid adduct were added, such that the final concentrations were 3, 10, 30, or 60 µM, respectively.

At the point of 24 hours after addition, formation of a mitochondrial respiratory chain supercomplex was detected by detecting FRET between GFP and RFP, resulting from the association of complex I and complex IV.

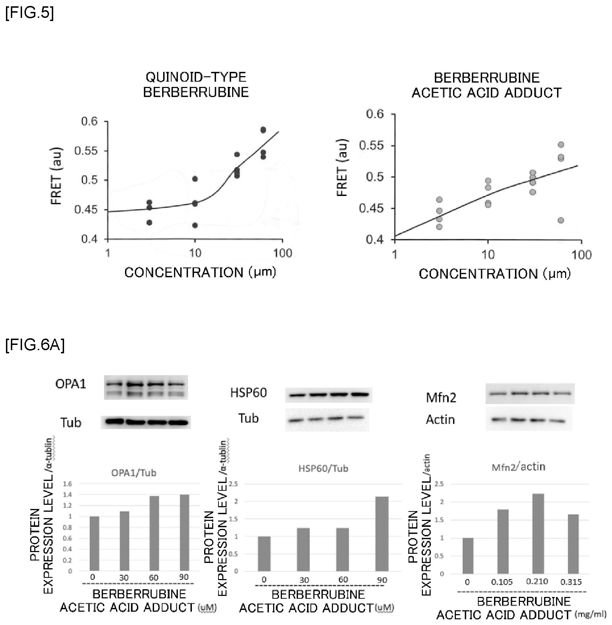

As shown in FIG. 5, the addition of quinoid-type berberrubine or berberrubine acetic acid adduct promoted the formation of mitochondrial respiratory chain supercomplexes in a concentration-dependent manner.

The formation of mitochondrial respiratory chain supercomplexes is known to enhance mitochondrial function by, for example, increasing the energy production amount by mitochondria and inhibiting the generation of reactive oxygen species (ROS) in the cells. That is, the above results indicate that the quinoid-type berberrubine and berberrubine acetic acid adduct improve mitochondrial function. Therefore, the quinoid-type berberrubine and berberrubine acetic acid adduct can provide an advantageous effect on diseases and symptoms caused by mitochondrial disorders. Moreover, the quinoid-type berberrubine and berberrubine acetic acid adduct can keep cells in a healthy state by mechanisms such as inhibiting the generation of reactive oxygen species (ROS), for example, to slow the progression of aging.

### [EXAMPLE 5]

### EFFECTS ON MOUSE PITUITARY CELLS

Pituitary glands removed from fetal C57BL/6NJcl mice were cut into pieces with scissors in an isolation buffer solution (0.123 M of sodium chloride, 5 mM of glucose, 5 mM of potassium chloride, 1.3 mM of calcium chloride, 100 mM of HEPES-NaOH (pH 7.4), 4% BSA) containing 1 mg/mL of collagenase, and warmed in a water bath at 37 °C for 30 minutes. After removal of aggregates using a cell strainer, the cells were collected by centrifugation at 1000 x g for 5 minutes and suspended in a DMEM medium containing 20% FBS, thereby preparing primary cultured cells of the pituitary cells.

3.5 mg/mL of an aqueous solution of the berberrubine acetic acid adduct was added to these primary culture cells such that the final concentrations were 0, 30, 60, or 90 µM.

After 48 hours of culturing after addition, mitochondrial proteins OPA1, HSP60, and Mfn2, as well as cellular protein Tub (α-tubulin) or Actin (actin) were detected by means of Western blotting.

As shown in FIG. 6A, the ratio of expression levels of mitochondrial proteins OPA1, HSP60, and Mfn2 with respect to cellular protein Tub or actin increased in a concentration-dependent manner with the berberrubine acetic acid adduct. The ratio of the expression level of OPA1 to Tub (OPA1/Tub), the ratio of the expression level of HSP60 to Tub (HSP60/Tub), and the ratio of the expression level of Mfn2 to Actin (Mfn2/Actin) each reflect the mitochondrial content in the cells. That is, these results show that the berberrubine acetic acid adduct increases the content of mitochondria in the cells in a concentration-dependent manner.

Meanwhile, the cell proliferation rate in the primary cultured cells to which the berberrubine acetic acid adduct had been added was comparable to that in the primary cultured cells to which the berberrubine acetic acid adduct had not been added.

Moreover, as shown in FIG. 6B, the primary cultured cells to which the berberrubine acetic acid adduct had been added had the nuclei stained with Hoechst and the mitochondria stained with Tom20, and were observed under a fluorescence microscope (magnification: 60x). No changes were observed compared to the primary cultured cells to which the berberrubine acetic acid adduct had not been added, nor were there any features observed in cancer cells or senescent cells that are not present in healthy cells, such as the nuclei being enlarged.

These results indicate that the berberrubine acetic acid adduct increases the content of mitochondria in the cells while not affecting healthy cell growth or morphology.

### [EXAMPLE 6]

### EFFECT ON PARKINSON'S DISEASE

Mouse fetal fibroblast (MEF) cells and PINK1-knockout MEF cells were cultured for 24 hours in a low glucose medium (manufactured by NACALAI TESQUE, INC.) containing 10% fetal bovine serum (FBS), penicillin, and streptomycin, and 3.5 mg/mL of a DMSO solution of quinoid-type berberrubine or 3.5 mg/mL of an aqueous solution of a berberrubine acetic acid adduct was added to the medium such that the final concentration was 30 µM. 48 hours after addition, Tom20, which is a mitochondrial outer membrane protein, was detected by immunofluorescence staining. For the detection of Tom20, the anti-Tom20 antibody (Santa Cruz, sc-11415) was used.

Note that PINK1 is the gene responsible for Parkinson's disease, and PINK1 deficiency causes mitochondrial dysfunction associated with Parkinson's disease. Therefore, PINK1-knockout MEF cells are used as a model for patients affected by Parkinson's disease.

As shown in FIG. 7, when the quinoid-type berberrubine or berberrubine acetic acid adduct was administered to the MEF cells, the content of mitochondria increased in the cells compared to the control (DMSO or water). Similarly, when the quinoid-type berberrubine or berberrubine acetic acid adduct was added to PINK1-knockout MEF cells, the content of mitochondria increased in the cells.

These results indicate that the quinoid-type berberrubine and berberrubine acetic acid adduct can inhibit mitochondrial dysfunction associated with Parkinson's disease by increasing the content of mitochondria in the cells. Therefore, the quinoid-type berberrubine and berberrubine acetic acid adduct can be used effectively in the treatment or prevention of Parkinson's disease.

### [EXAMPLE 7]

### INHIBITORY EFFECT ON CANCER CELL GROWTH

3.5 mg/mL of a DMSO solution of quinoid-type berberrubine or 3.5 mg/mL of an aqueous solution of a berberrubine acetic acid adduct was added to HeLa cells in Dulbecco's modified Eagle (DMEM) medium containing 10% fetal bovine serum (FBS), penicillin, and streptomycin such that the final concentration was 30 µM. Cells were collected at 24, 48, and 72 hours after addition and stained with trypan blue, and the number of cells were measured. Note that the HeLa cells are cell lines derived from human uterus cancer cells and are widely used as model cells for cancer.

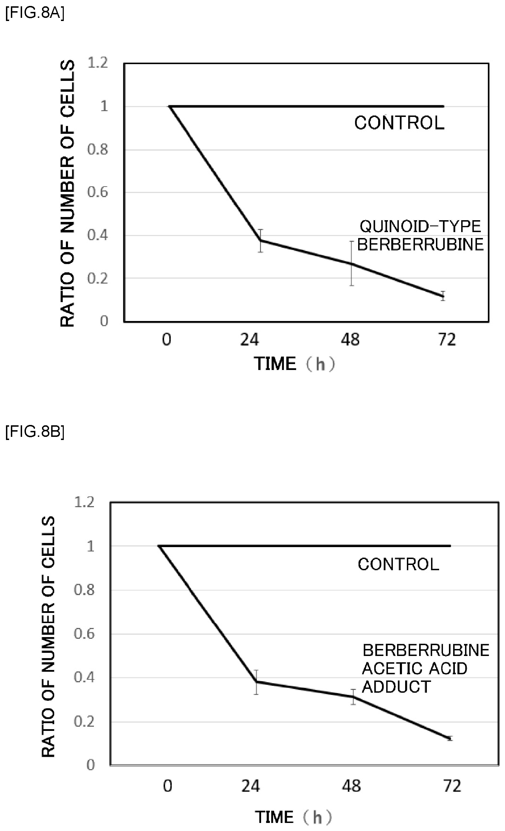

As shown in FIG. 8A, the ratio of the number of HeLa cells after administration of quinoid-type berberrubine with respect to the number of HeLa cells in the control decreased over time. That is, the addition of quinoid-type berberrubine reduced the growth rate of the HeLa cells compared to the control (DMSO). Moreover, as shown in FIG. 8B, the ratio of the number of HeLa cells after administration of a berberrubine acetic acid adduct with respect to the number of HeLa cells in the control decreased over time. That is, the addition of an aqueous solution of a berberrubine acetic acid adduct reduced the growth rate of the HeLa cells compared to the control (water).

These results indicate that the quinoid-type berberrubine and berberrubine acetic acid adduct have an effect of inhibiting cancer cell growth and are effective in the treatment or prevention of cancer.

### [EXAMPLE 8]

### EFFECT ON MYELODYSPLASTIC SYNDROME (MDS)

A retroviral vector incorporating co-introduced clones of CBL mutant and RNUX1 mutant was infected with c-Kit+ cells recovered from 7-week-old mice. These cells were transplanted through the tail vein into 7-week-old mice irradiated with sublethal doses of radiation, thereby creating hematopoietic stem cell transplanted (BMT) mice with myelodysplastic syndrome (MDS).

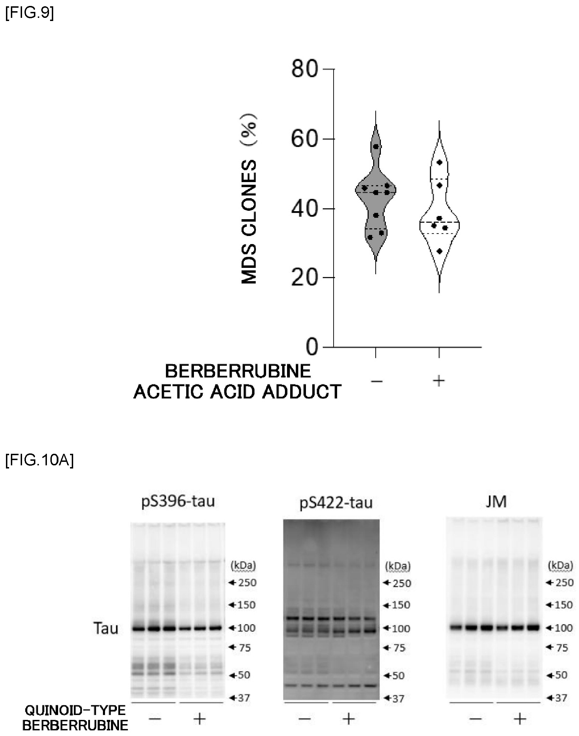

As shown in FIG. 9, the BMT mice to which a berberrubine acetic acid adduct was administered had a significantly reduced ratio (P < 0.01) of MDS clones (co-introduced clones of CBL mutant and RNUX1 mutant) with respect to the number of blood cells compared to the control.

These results indicate that the berberrubine acetic acid adduct is effective in the treatment or prevention of cancer including MDS.

### [EXAMPLE 9]

### EFFECTS ON EXPRESSION AND PHOSPHORYLATION OF NEUROLOGICAL DISEASE-RELATED PROTEINS

Neuro2A cells were introduced with ΔN-P301Ltau-CRY2olig by transfection. One day after introduction, nocodazole was added to stop the cell cycle, and subsequent blue light irradiation for 24 hours induced the aggregation of tau proteins in the cells. 3.5 mg/mL of a DMSO solution of quinoid-type berberrubine or 3.5 mg/mL of an aqueous solution of a berberrubine acetic acid adduct was added to Neuro2A cells with aggregated tau proteins such that the final concentration is 30 µM.

After 8 hours of culturing after addition, the tau proteins in the cells were detected by means of Western blotting using an antibody against phosphorylated Tau at position S396 (pS396), an antibody against phosphorylated Tau at position S422 (pS422), an antibody against full length Tau (JM), and an antibody against the C-terminal region of Tau (tauC). The tau proteins were detected at around 100 kDa.

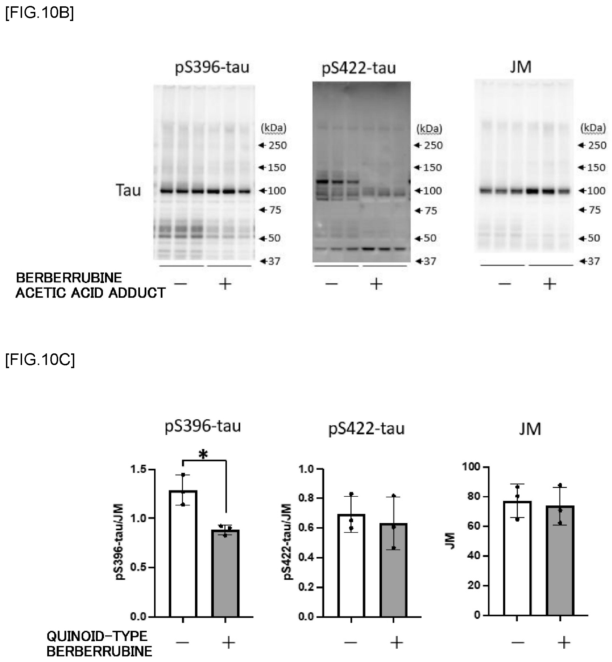

As shown in FIG. 10A, the addition of quinoid-type berberrubine decreased the expression level of phosphorylated Tau at position S396. More specifically, as shown in FIG. 10C, the addition of quinoid-type berberrubine reduced the ratio of the expression level of phosphorylated Tau at position S396 with respect to that of full-length Tau (left graph in FIG. 10C (vertical axis "pS396-tau/JM")) by about 35% (p<0.01) compared to the control (to which only DMSO was administered). Meanwhile, no significant changes were observed for the ratio of the expression level of phosphorylated Tau at position S422 with respect to that of full-length Tau (middle graph in FIG. 10C (vertical axis "pS422-tau/JM")). Moreover, no significant changes were observed in the expression level of full-length Tau (right graph in FIG. 10C).

Phosphorylation at position S396 of tau protein is known to be caused by GSK3β. That is, these results indicate that the addition of the quinoid-type berberrubine inhibited phosphorylation of tau proteins by GSK3β.

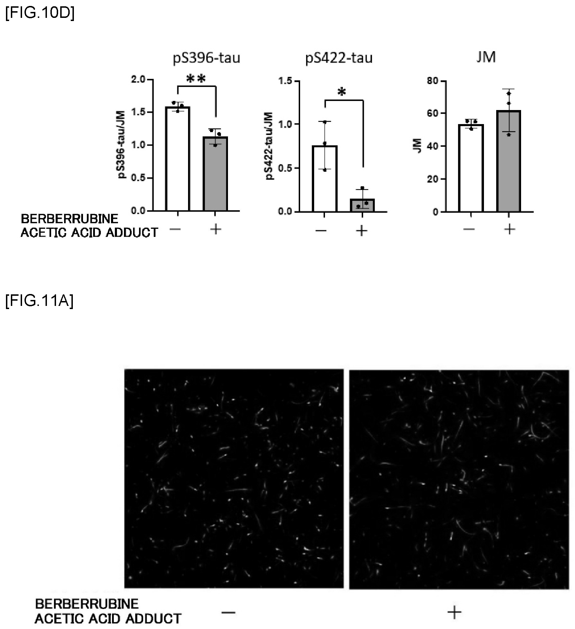

As shown in FIG. 10B, the addition of a berberrubine acetic acid adduct decreased the expression level of phosphorylated Tau at position S422. More specifically, as shown in FIG. 10D, the addition of a berberrubine acetic acid adduct reduced the ratio of the expression level of phosphorylated Tau at position S422 with respect to that of full-length Tau (middle graph in FIG. 10D (vertical axis "pS422-tau/JM")) by about 35% (p<0.01) compared to the control (to which only water was administered). Meanwhile, no significant changes were observed for the ratio of the expression level of phosphorylated Tau at position S396 with respect to that of full-length Tau (left graph in FIG. 10D (vertical axis "pS396-tau/JM")). Moreover, no significant changes were observed in the expression level of full-length Tau (right graph in FIG. 10C).

Phosphorylation at position S422 of tau protein is known to be caused by JNK. That is, these results indicate that the addition of the berberrubine acetic acid adduct inhibited the phosphorylation of tau proteins by JNK.

Both phosphorylation of tau proteins by GSK3β and phosphorylation of tau proteins by JNK are known to cause neurological diseases, such as dementia (especially Alzheimer's disease). Therefore, both the quinoid-type berberrubine and berberrubine acetic acid adduct can provide advantageous effects in the treatment or prevention of neurological diseases.

Moreover, the berberrubine acetic acid adduct used herein is an acetic acid adduct of enol-type berberrubine and/or zwitterionic-type berberrubine, as described above. Moreover, the acetic acid (or acetate ions) contained in the berberrubine acetic acid adduct is released in the sample (in water), so the berberrubine acetic acid adduct acts as enol-type berberrubine and/or zwitterionic-type berberrubine. Therefore, compared to the enol-type berberrubine and/or zwitterionic-type berberrubine, the quinoid-type berberrubine can provide a more advantageous effect on diseases and symptoms caused by mitochondrial disorders.

Meanwhile, the quinoid-type berberrubine and berberrubine acetic acid adduct (that is, enol-type berberrubine and/or zwitterionic-type berberrubine) have different mechanisms of inhibition of phosphorylation of tau proteins, and thus are considered to have different mechanisms of action. More specifically, the quinoid-type berberrubine is considered to inhibit the activity of GSK3β, and the berberrubine acetic acid adduct (that is, enol-type berberrubine and/or zwitterionic-type berberrubine) is considered to inhibit the activity of JNK.

### [EXAMPLE 10]

### EFFECT ON SPERM

Male C57BL/6NJcl mice aged 9 to 10 weeks were orally administered 1.25% agar containing 0.35 mg/mL of quinoid-type berberrubine or 1.25% agar not containing quinoid-type berberrubine for 2 weeks. The oral administration was performed by placing a container containing this agar in a cage with the mice, such that the mice could constantly ingest this agar from the container (subsequent oral administration of agar to mice was performed in the same manner). At 20 weeks of age, the mice were anesthetized isoflurane (KANTO KAGAKU), euthanized by cervical dislocation, and had their testes and epididymis removed. The removed epididymis was cut into pieces with scissors in 1 mL of phosphate buffered saline (PBS) and then placed at room temperature for 1 hour, thereby preparing sperm samples. 20 µL of the sample was transferred to a glass bottom dish, and mitochondria and nuclei were stained by MitoTracker (manufactured by Thermo Fisher Scientific) and Hoechst (manufactured by NACALAI TESQUE, INC.), respectively.

Observation with a fluorescence microscope revealed that sperm were more actively motile in mice treated with quinoid-type berberrubine compared to the control. The observation results were recorded as videos. FIG. 11A is a snapshot of a recorded video. Stained sperm, shown in FIG. 11A, were more actively motile in mice to which quinoid-type berberrubine was administered, compared to the control.

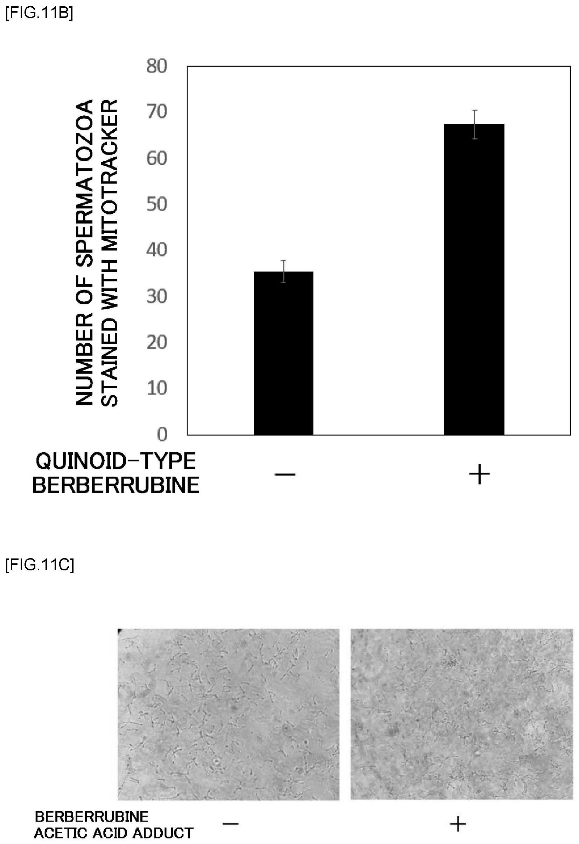

Moreover, observation with a fluorescence microscope revealed that the number of spermatozoa in which mitochondria were stained with MitoTracker in mice administered quinoid-type berberrubine increased about twofold compared to the control (FIG. 11B). MitoTracker is a detection reagent that relies on mitochondrial membrane potential to detect mitochondria. Moreover, the enhancement of mitochondrial membrane potential is known to be due to the activation of mitochondrial function. Therefore, these results indicate that the quinoid-type berberrubine activated mitochondrial function.

Similarly, even in mice orally administered 2 mg/mL of an aqueous solution of a berberrubine acetic acid adduct, it was observed that spermatozoa were more actively motile comparted to the control. The observation results were recorded as videos. FIG. 11C is a snapshot of a recorded video.

These results indicate that the quinoid-type berberrubine and berberrubine acetic acid adduct enhance the motility of spermatozoa and sperm mitochondrial function. Therefore, the quinoid-type berberrubine and berberrubine acetic acid adduct can provide advantageous effects in the treatment or prevention of infertility.

### [EXAMPLE 11]

### EFFECT ON SKIN

Male C57BL/6NJcl mice aged 8 weeks were orally administered 1.25% agar containing 0.35 mg/mL of quinoid-type berberrubine or 1.25% agar not containing quinoid-type berberrubine (control) for 2 months. The skin of the mice during the same period was irradiated with UV 3 times a week for 8 weeks. The irradiation dose was 1 mW/cm² for 70 seconds (70 mJ/cm²) in the first week, 1 mW/cm² for 80 seconds (80 mJ/cm²) in the second week, 1 mW/cm² for 90 seconds (90 mJ/cm²) in the third week, and 1 mW/cm² for 100 seconds (100 mJ/cm²) in the fourth week and thereafter. At the end of the same period, the skin of the mice was visually observed.

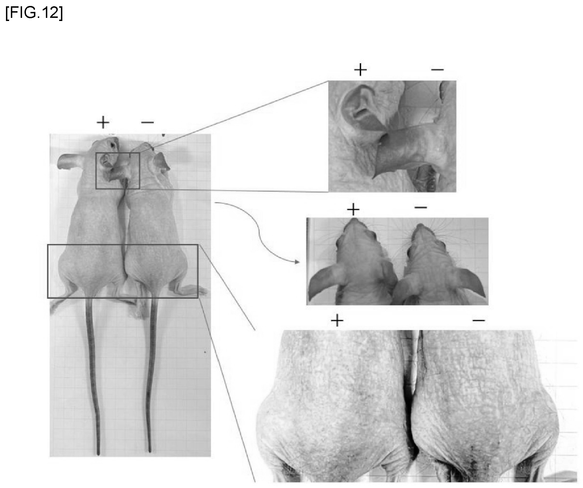

As shown in FIG. 12, the mice to which quinoid-type berberrubine was administered had fewer wrinkles and shallower wrinkle depths than the mice to which quinoid-type berberrubine was not administered. Moreover, the skin darkening of the mice to which the quinoid-type berberrubine was administered was inhibited compared to the mice to which the quinoid-type berberrubine was not administered.

These results indicate that the quinoid-type berberrubine inhibits the formation of wrinkles in the skin, reduces the number and depth of wrinkles, and inhibits skin darkening.

### [EXAMPLE 12]

### EFFECT ON VISCERAL FAT REDUCTION

Male C57BL/6NJcl mice aged 50 weeks were orally administered 1.25% agar containing 0.35 mg/mL of quinoid-type berberrubine or 1.25% agar not containing quinoid-type berberrubine (control) for 2 months. At the end of the period, accumulation of visceral fat in the mice was observed.

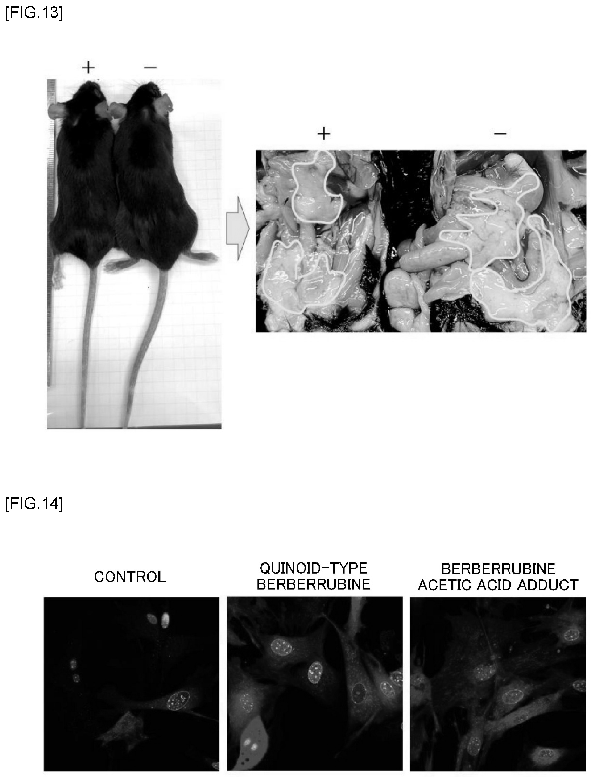

As shown in FIG. 13, visceral fat accumulation of the mice to which quinoid-type berberrubine was administered was inhibited compared to the mice to which quinoid-type berberrubine was not administered.

These results indicate that the quinoid-type berberrubine can inhibit the accumulation of body fat, including visceral fat, and provide an advantageous effect in the amelioration or prevention of obesity.

### [EXAMPLE 13]

### EFFECT ON BROWN FAT CELLS

Brown fat cells removed from fetal C57BL/6NJcl mice were cut into pieces with scissors in an isolation buffer solution (0.123 M of sodium chloride, 5 mM of glucose, 5 mM of potassium chloride, 1.3 mM of calcium chloride, 100 mM of HEPES-NaOH (pH 7.4), 4% BSA) containing 1 mg/mL of collagenase, and warmed in a water bath at 37 °C for 30 minutes. After removal of aggregates using a cell strainer, the cells were collected by centrifugation at 1000 x g for 5 minutes and suspended in a DMEM medium containing 20% FBS, thereby preparing primary cultured cells of the brown fat cells.

3.5 mg/mL of a DMSO solution of quinoid-type berberrubine or 3.5 mg/mL of an aqueous solution of a berberrubine acetic acid adduct was added to these primary cultured cells such that the final concentration was 30 µM. Moreover, as a control, the same amount of DMSO as in the DMSO solution of quinoid-type berberrubine was added to the primary cultured cells.

The primary cultured cells cultured for 48 hours after addition had the nuclei stained with Hoechst (manufactured by NACALAI TESQUE, INC.) and mitochondria stained with tetramethylrhodamine methyl ester (TMRM, manufactured by Thermo Fisher Scientific) and then were observed under a fluorescence microscope (magnification: 60x).

As shown in FIG. 14, strong staining of mitochondria by TMRM was observed with the primary cultured cells to which the quinoid-type berberrubine and berberrubine acetic acid adduct had been added.

TMRM is a cell-penetrating fluorescent dye that accumulates in active mitochondria having normal membrane potential, and the signal is brighter when the cells are healthy and have functioning mitochondria. Therefore, these results indicate that the quinoid-type berberrubine and berberrubine acetic acid adduct increase active mitochondria having normal membrane potential in brown fat cells.

Moreover, as shown in FIG. 14, features observed in cancer cells or senescent cells that are not present in healthy cells, such as the nuclei being enlarged, were not observed in the primary cultured cells to which the quinoid-type berberrubine or berberrubine acetic acid adduct had been added, similar to the control.

The above results indicate that the quinoid-type berberrubine and berberrubine acetic acid adduct provide an advantageous effect in diseases and symptoms caused by mitochondrial disorders by increasing active mitochondria.

Moreover, activation of mitochondrial function in brown fat cells is also known to reduce white fat cells such as subcutaneous fat. Therefore, the above results indicate that the berberrubine and berberrubine acetic acid adduct provide an advantageous effect in the amelioration or prevention of obesity.

In addition, as shown in FIG. 14, the addition of quinoid-type berberrubine increased the amount of active mitochondria having normal membrane potential to a greater extent compared to the addition of a berberrubine acetic acid adduct.

Therefore, the quinoid-type berberrubine can provide a more advantageous effect compared to the berberrubine acetic acid adduct for diseases and symptoms caused by mitochondrial disorders or for amelioration or prevention of obesity.

Moreover, the berberrubine acetic acid adduct used herein is an acetic acid adduct of enol-type berberrubine and/or zwitterionic-type berberrubine as described above. Moreover, acetic acid (or acetate ions) is released from enol-type berberrubine and/or zwitterionic-type berberrubine in a sample (in water). Therefore, compared to the enol-type berberrubine and/or zwitterionic-type berberrubine, the quinoid-type berberrubine can provide a more advantageous effect for diseases and symptoms caused by mitochondrial disorders or in amelioration or prevention of obesity.

### [EXAMPLE 14]

### EFFECT ON GROWTH OF BEAN SPROUTS

Seeds of bean sprouts were sown in a 10 cm petri dish containing 0.035 mg/mL of an aqueous solution of a berberrubine acetic acid adduct or water, and the growth of the bean sprouts was observed every day.

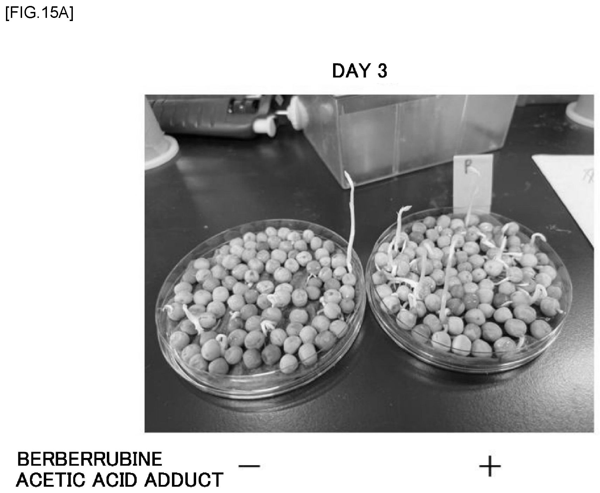

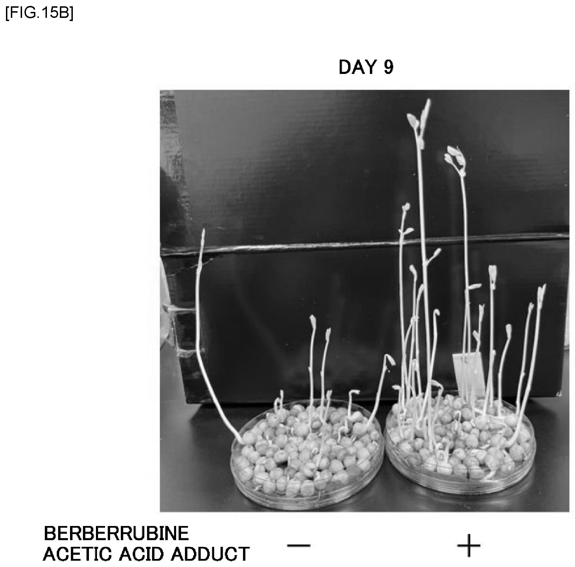

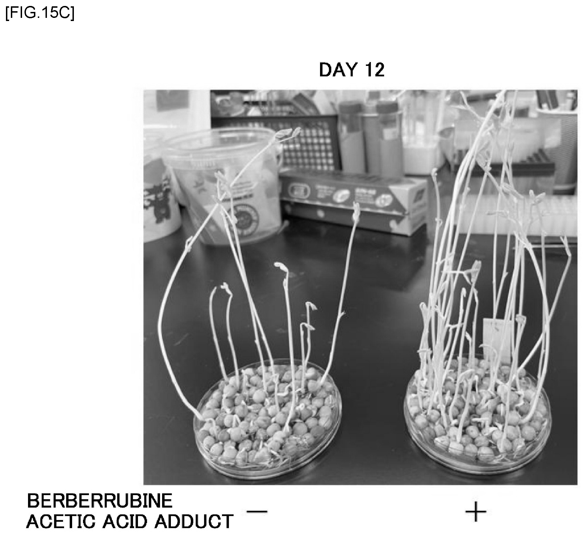

As shown in FIG. 15, the bean sprouts grown in the aqueous solution of the berberrubine acetic acid adduct solution had more sprouting and more rapid subsequent growth than those grown in water.

These results indicate that the berberrubine acetic acid adduct has an effect of promoting plant growth and is useful as an agricultural composition.

### [EXAMPLE 15]

### EFFECT ON HEART (1)

A berberrubine acetic acid adduct was administered to mice by allowing C57BL/6N24 mice from 24 months of age onwards to freely ingest 0.5 mg/mL of an aqueous solution of a berberrubine acetic acid adduct or water (control) as drinking water.

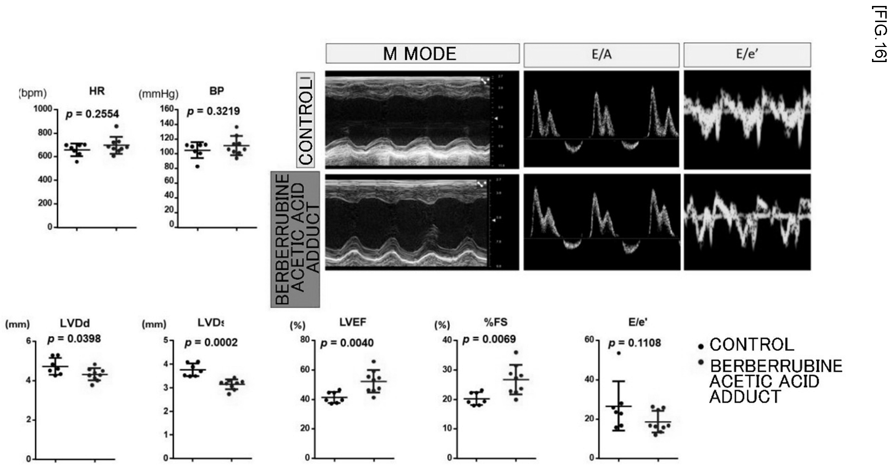

The results of echocardiography using the M-mode echocardiography technique on the mice 12 weeks after the start of administration are shown in FIG. 16.

As shown in FIG. 16, individuals to which a berberrubine acetic acid adduct was administered, compared to the control, had a decrease in left ventricular end-diastolic diameter (LVDd), left ventricular end-systolic diameter (LVDs), left ventricular diastolic function, and left ventricular filling pressure (E/e'), and had an increase in left ventricular ejection fraction (LVEF) and % left ventricular fractional shortening (%FS). These results indicate that the berberrubine acetic acid adduct effectively improves cardiac function.

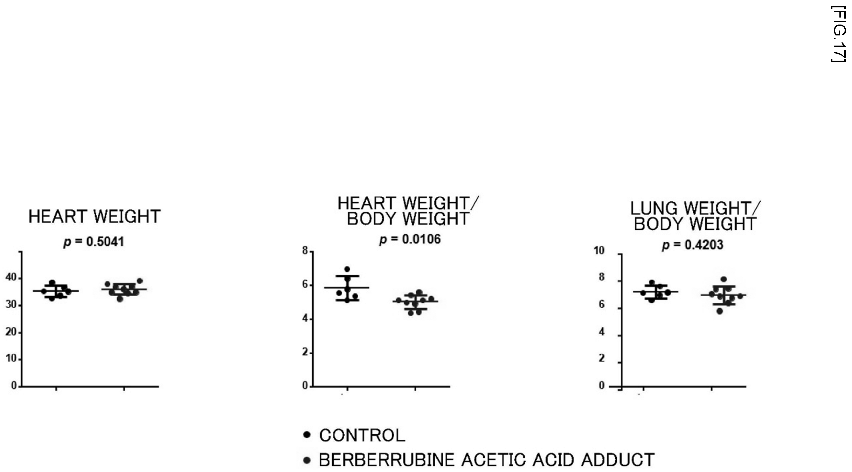

Moreover, the autopsy report for the mice 12 weeks after the start of administration is also shown in FIG. 17.

As shown in FIG. 17, individuals to which a berberrubine acetic acid adduct was administered had a decreased ratio of heart weight with respect to body weight compared to the control. Meanwhile, no significant change was seen in the ratio of lung weight with respect to body weight.

These results indicate that the berberrubine acetic acid adduct is effective in ameliorating and/or preventing cardiac hypertrophy.

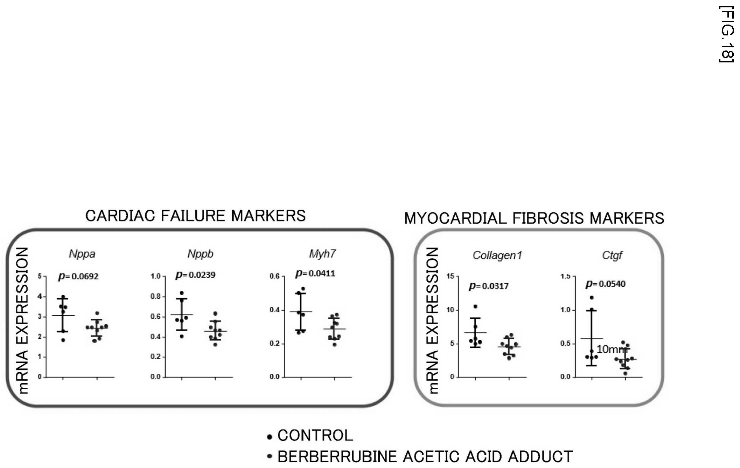

Moreover, the results of quantifying cardiac failure and myocardial fibrosis markers in the mice 12 weeks after the start of administration by means of quantitative RT-PCR are shown in FIG. 18.

As shown in FIG. 18, the expression levels of cardiac failure markers (Nppa, Nppb, and Myh7) and myocardial fibrosis markers (Collagen1 and Ctgf) were reduced in individuals administered a berberrubine acetic acid adduct as compared to the control.

These results indicate that the berberrubine acetic acid adduct effectively reduces the risk of cardiac failure and myocardial fibrosis.

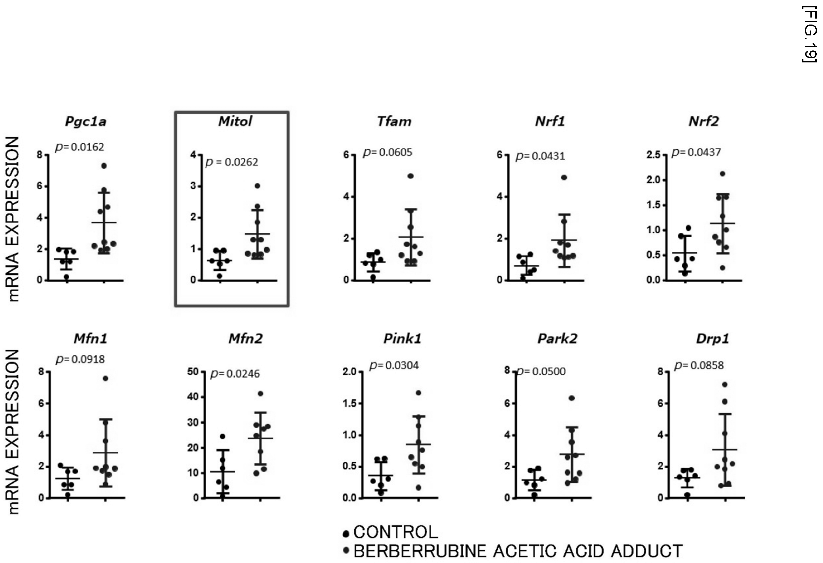

Moreover, the results of quantifying the mRNA expression of mitochondria in the mice 12 weeks after the start of administration by means of quantitative RT-PCR are shown in FIG. 19.

As shown in FIG. 19, the expression levels of mitochondrial mRNAs (Pgc1a, Mitol, Tfam, Nrf1, Nrf2, Mfn1, Mfn2, Pink1, Park2, and Drp1) were increased in the individuals administered a berberrubine acetic acid adduct as compared to the control.

These results indicate that the berberrubine acetic acid adduct activates mitochondria in the heart.

The above results indicate that the berberrubine acetic acid adduct is effective in maintaining normal cardiac function or ameliorating cardiac function, ameliorating or preventing cardiac hypertrophy, and treating and/or preventing cardiac disease.

### [EXAMPLE 16]

### EFFECT ON HEART (2)

C57BL/6N24 mice from 24 months of age onwards were administered the following drinking water and food.
(1) Normal water as drinking water and a normal diet (CE-2, CLEA Japan, Inc., hereafter the same) as food (control).
(2) 0.5 mg/mL of an aqueous solution of a berberrubine acetic acid adduct as drinking water and a normal diet as food (administration of a berberrubine acetic acid adduct).
(3) Normal water as drinking water and a normal diet containing 0.5 mg/mL of quinoid-type berberrubine as food (administration of quinoid-type berberrubine).

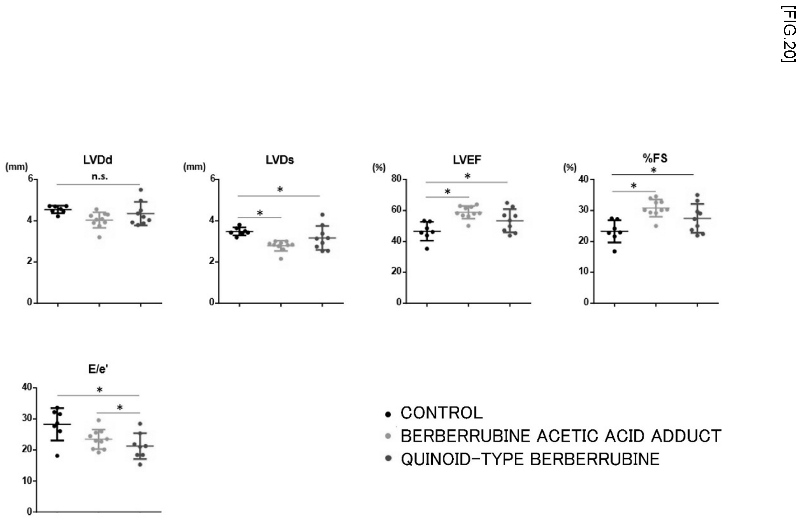

The results of echocardiography using the M-mode echocardiography technique on the mice 12 weeks after the start of administration are shown in FIG. 20.

As shown in FIG. 20, both individuals to which a berberrubine acetic acid adduct and quinoid-type berberrubine were administered, compared to the control, had a decrease in left ventricular end-diastolic diameter (LVDd), left ventricular end-systolic diameter (LVDs), and a measure of left ventricular filling pressure (E/e'), and had an increase in left ventricular ejection fraction (LVEF) and % left ventricular fractional shortening (%FS).

These results indicate that the berberrubine acetic acid adduct and quinoid-type berberrubine effectively improve cardiac function.

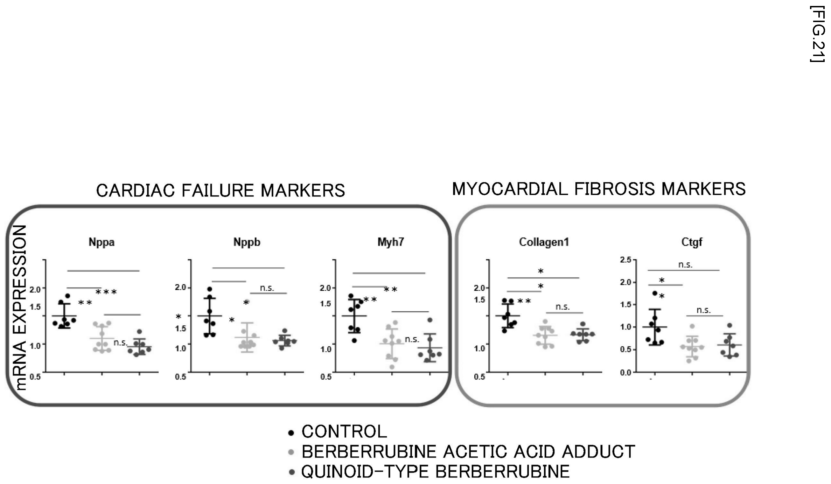

Moreover, the results of quantifying cardiac failure and myocardial fibrosis markers in the mice 12 weeks after the start of administration by means of quantitative RT-PCR are shown in FIG. 21.

As shown in FIG. 21, the expression levels of cardiac failure markers (Nppa, Nppb, and Myh7) and myocardial fibrosis markers (Collagen1 and Ctgf) were reduced in individuals to which a berberrubine acetic acid adduct and quinoid-type berberrubine were administered as compared to the control.

These results indicate that the berberrubine acetic acid adduct and quinoid-type berberrubine effectively reduce the risk of cardiac failure and myocardial fibrosis.

Moreover, the results of quantifying the expression of Mitol which is mRNA of mitochondria in the mice 12 weeks after the start of administration by means of quantitative RT-PCR are shown in FIG. 22.

As shown in FIG. 22, both individuals to which a berberrubine acetic acid adduct and quinoid-type berberrubine were administered had an increase in the expression of Mitol as compared to the control.

These results indicate that the berberrubine acetic acid adduct and quinoid-type berberrubine effectively activate mitochondria in the heart.

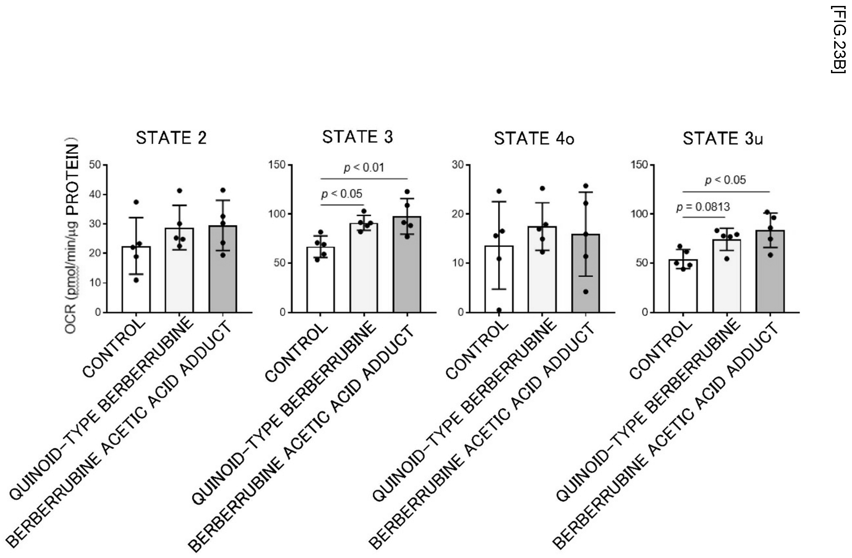

Moreover, mitochondria were isolated from cardiomyocytes of mice 12 weeks after the start of administration using a standard method. The results of measuring the oxygen consumption rate (OCR) using a flux analyzer are shown in FIGs. 23A and 23B.

As shown in FIGs. 23A and 23B, the berberrubine acetic acid adduct and quinoid-type berberrubine exhibited increased oxygen consumption rates (OCRs) in State 2 (ADP-limited state; indicating basic mitochondrial respiratory rate), State 3 (activated respiratory state; indicating mitochondrial energy-producing capacity), State 4o (oligomycin-induced state; indicating non-phosphorylating oxygen consumption (proton leakage) of mitochondria), and State 3u (uncoupled state; indicating maximum oxygen consumption capacity of mitochondria), compared to the control. These results indicate that the berberrubine acetic acid adduct and quinoid-type berberrubine effectively activate mitochondria in the heart.

The above results indicate that the berberrubine acetic acid adduct and quinoid-type berberrubine are effective in maintaining normal cardiac function or ameliorating cardiac function, ameliorating or preventing cardiac hypertrophy, and treating and/or preventing cardiac disease.

### [EXAMPLE 17]

### EFFECT ON MITOCHONDRIAL ENCEPHALOMYOPATHY, LACTIC ACIDOSIS, AND STROKE-LIKE EPISODE (MELAS)

For human dermal fibroblast (NHDF) cells and mitochondrial encephalomyopathy, lactic acidosis, and stroke-like episode (MELAS) derived cells F954 (3243A>G, 92%) and F969 (3243A>G, 34.5%), the analysis start day was set to day 0. On day 1, an aqueous solution of a berberrubine acetic acid adduct solution or water with a final concentration of 30 µM was added to a cell culture medium, and on day 8, the oxygen consumption rate (OCR) was measured by a flux analyzer.

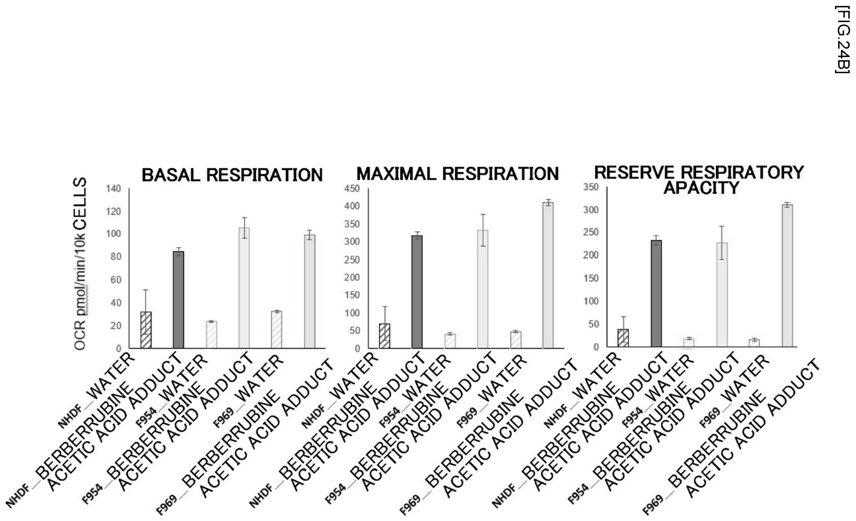

As shown in FIGs. 24A and 24B, when an aqueous solution of a berberrubine acetic acid adduct is added, basal, maximal, and reserve respiratory capacity of NHDF, F954, and F969 cells are increased compared to when water was added, indicating enhanced mitochondrial function. The degree of increase was greater in F954 and F969, which are MERAS-derived cells, compared to NHDF cells.

These results indicate that the berberrubine acetic acid adduct is effective in activating mitochondria, and particularly in the treatment and/or prevention of MERAS.

### [EXAMPLE 18]

### EFFECT ON INFLAMMATORY CANCER

The effects of the berberrubine acetic acid adduct on inflammatory cancer were analyzed using a mouse inflammation-associated carcinogenesis model.

That is, a berberrubine acetic acid adduct was administered to mice by allowing 57BL/6 mice from 5 weeks of age onwards to freely ingest 1 mg/mL of an aqueous solution of a berberrubine acetic acid adduct or water as drinking water.

On day 7 from the start of administration, regressive-type QR-32 cells and gelatin sponges were co-transplanted into C57BL/6 mice subcutaneously on the right side of the mouse body according to the method described in Okada et al., Br. J. Cancer, volume 66, pages 635-639 (1992), thereby forming a tumor.

The size of the tumors formed in the mice after co-transplantation was observed.

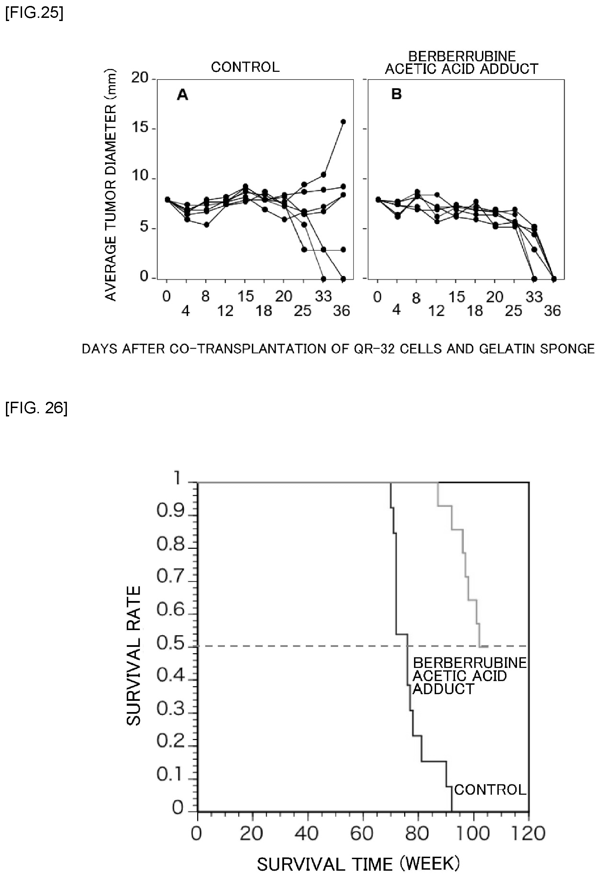

As shown in FIG. 25, 36 weeks after co-transplantation, tumors remained in 5 out of 7 mice (71%) that ingested water as drinking water. In contrast, 36 weeks after co-transplantation, none of the mice that ingested an aqueous solution of a berberrubine acetic acid adduct as drinking water had tumors (0 out of 6 mice (0%)).

These results indicate that the berberrubine acetic acid adduct effectively inhibits inflammatory cancers.

Note that it is possible to perform a similar analysis of the effect of quinoid-type berberrubine on inflammatory cancers.

### [EXAMPLE 19]

### EFFECT ON EXTENDING LIFESPAN

Mice from 8 weeks of age onwards were allowed to ingest a high-fat diet (High Fat Diet 32; CLEA Japan, Inc.) as food, and from 60 weeks of age onward, were also allowed to freely ingest 0.5 mg/mL of an aqueous solution of a berberrubine acetic acid adduct or water (control) as drinking water, and the survival time was analyzed.

As shown in FIG. 26, administering the berberrubine acetic acid adduct extended survival time compared to the control. That is, regarding the control individuals, all 13 individuals that were tested at 92 weeks of age died (survival rate: 0%). Meanwhile, regarding the individuals to which the berberrubine acetic acid adduct was administered, 13 out of the 14 individuals tested at 92 weeks of age were alive (survival rate: 93%).

Moreover, a 50% survival rate was obtained with control individuals at 75 weeks of age, whereas a 50% survival rate was obtained with the individuals to which the berberrubine acetic acid adduct was administered at 105 weeks of age. That is, the berberrubine acetic acid adduct extended the 50% survival rate by 30 weeks.

These above results indicate that the berberrubine acetic acid adduct effectively extends lifespan.

The disclosed content of the patents, patent applications, and publications cited in the present disclosure are hereby incorporated by reference in their entirety.

### INDUSTRIAL APPLICABILITY

The composition of the present disclosure can be suitably used, for example, as pharmaceutical industrial products, food industrial products, cosmetic industrial products, or agricultural industrial products. Therefore, the present invention is industrially applicable.

## Claims

1. A composition comprising quinoid-type berberrubine represented by the following Formula (I)

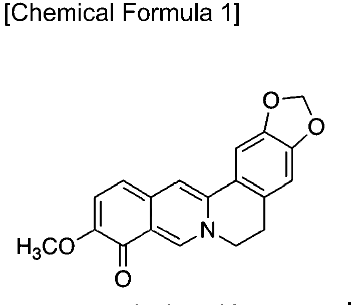

2. The composition according to claim 1, wherein the compound represented by Formula (I) is isolated quinoid-type berberrubine.

3. The composition according to claim 1, wherein the composition is a pharmaceutical composition.

4. The composition according to claim 1, wherein the composition is a food composition.

5. The composition according to claim 1, wherein the composition is a cosmetic composition.

6. The composition according to claim 1, wherein the composition is an agricultural composition.

7. The composition according to claim 1, wherein the composition is for activating mitochondria.

8. The composition according to claim 1, wherein the composition is for treating or preventing a mitochondrial disease.

9. The composition according to claim 1, wherein the composition is for treating or preventing Parkinson's disease.

10. The composition according to claim 1, wherein the composition is for treating or preventing mitochondrial encephalomyopathy, lactic acidosis, and stroke-like episodes (MELAS).

11. The composition according to claim 1, wherein the composition is for treating or preventing a cancer.

12. The composition according to claim 11, wherein the cancer is epithelial carcinoma, carcinoma, cervical cancer, sarcoma, or myelodysplastic syndrome (MDS).

13. The composition according to claim 1, wherein the composition is for treating or preventing a muscle disease.

14. The composition according to claim 13, wherein the muscle disease is frailty, locomotive syndrome, or sarcopenia.

15. The composition according to claim 1, wherein the composition is for treating or preventing a neurological disease.

16. The composition according to claim 15, wherein the neurological disease is dementia.

17. The composition according to claim 1, wherein the composition is for treating or preventing a heart disease.

18. The composition according to claim 17, wherein the cardiac disease is cardiac failure.

19. The composition according to claim 17, wherein the cardiac disease is cardiomyopathy.

20. The composition according to claim 1, wherein the composition is for ameliorating or preventing cardiac hypertrophy.

21. The composition according to claim 1, wherein the composition is for ameliorating infertility.

22. The composition according to claim 1, wherein the composition is for activating sperm activity.

23. The composition according to claim 1, wherein the composition is for preventing formation of wrinkles in skin, for reducing a depth and/or number of wrinkles, and/or for inhibiting an increase in the depth and/or number of wrinkles.

24. The composition according to claim 1, wherein the composition is for inhibiting or preventing darkening of skin.

25. The composition according to claim 1, wherein the composition is for ameliorating or preventing obesity.

26. The composition according to claim 1, wherein the composition is for slowing progression of aging.

27. The composition according to claim 1, wherein the composition is for extending lifespan.

28. The composition according to claim 1, wherein the composition is for promoting plant growth.

29. A production method of a berberrubine acetic acid adduct, the method comprising:
adding acetic acid to quinoid-type berberrubine represented by the following Formula (I)

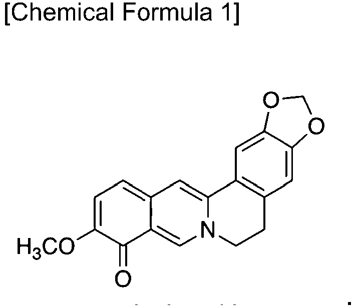

30. The production method according to claim 29, comprising adding acetic acid as acetic acid vapor.

31. A production method of an aqueous solution of a berberrubine acetic acid adduct, the method comprising:
(i) adding acetic acid to quinoid-type berberrubine represented by the following Formula (I) to produce a berberrubine acetic acid adduct: 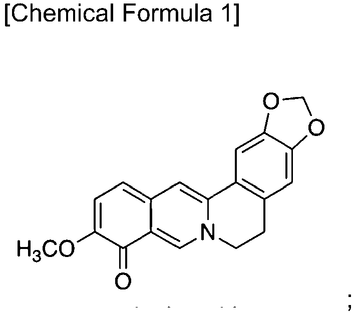 and
(ii) dissolving the produced berberrubine acetic acid adduct in water.

32. The production method according to claim 31, wherein in (i), acetic acid is added as acetic acid vapor to produce a berberrubine acetic acid adduct.

33. A berberrubine acetic acid adduct.

34. The berberrubine acetic acid adduct according to claim 33, wherein acetic acid and/or acetic acid ions are added to:
enol-type berberrubine represented by the following Formula (2): 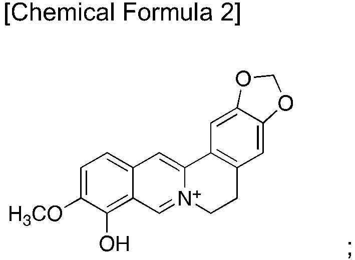 or
zwitterionic-type berberrubine represented by the following Formula (3):

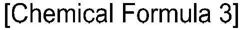

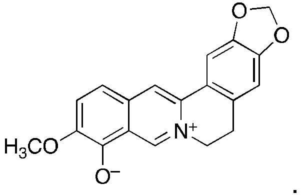

35. The berberrubine acetic acid adduct according to claim 33, wherein 1 equivalent or more of acetic acid and/or acetate ions are added to 1 equivalent of berberrubine.

36. The berberrubine acetic acid adduct according to claim 33, wherein the berberrubine acetic acid adduct is:
(i) a compound represented by the following Formula (4): 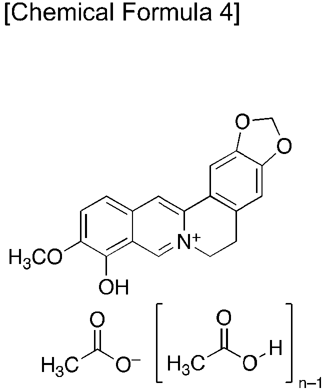 [wherein, n is 1 or more];
(ii) a compound represented by the following Formula (5): 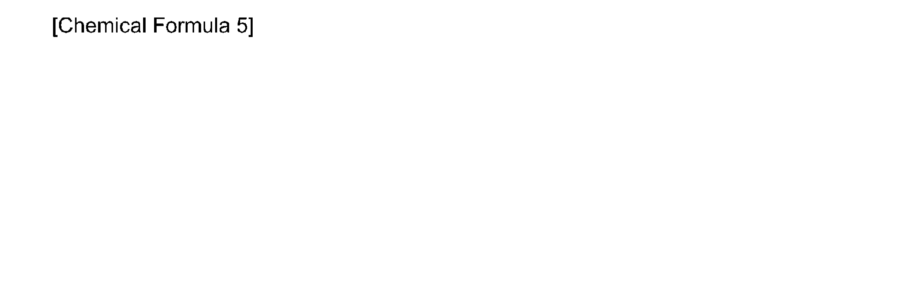 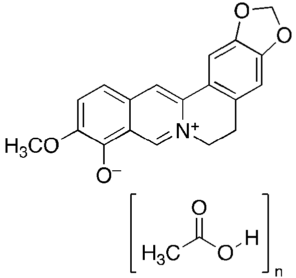 [wherein, n is 1 or more]; or
(iii) a mixture of the compound (i) and the compound (ii).

37. The berberrubine acetic acid adduct according to claim 33, wherein a solubility in water is 0.01 g/mL or more.

38. An aqueous solution of the berberrubine acetic acid adduct according to claim 33.

39. A composition comprising the berberrubine acetic acid adduct according to claim 33 or the aqueous solution according to claim 38.

40. The composition according to claim 39, wherein the composition is a pharmaceutical composition.

41. The composition according to claim 39, wherein the composition is a food composition.

42. The composition according to claim 39, wherein the composition is a cosmetic composition.

43. The composition according to claim 39, wherein the composition is an agricultural composition.

44. The composition according to claim 39, wherein the composition is for activating mitochondria.

45. The composition according to claim 39, wherein the composition is for treating or preventing a mitochondrial disease.

46. The composition according to claim 39, wherein the composition is for treating or preventing Parkinson's disease.

47. The composition according to claim 39, wherein the composition is for treating or preventing mitochondrial encephalomyopathy, lactic acidosis, and stroke-like episodes (MELAS).

48. The composition according to claim 39, wherein the composition is for treating or preventing a cancer.

49. The composition according to claim 48, wherein the cancer is epithelial carcinoma, carcinoma, cervical cancer, sarcoma, or myelodysplastic syndrome (MDS).

50. The composition according to claim 39, wherein the composition is for treating or preventing a muscle disease.

51. The composition according to claim 50, wherein the muscle disease is frailty, locomotive syndrome, or sarcopenia.

52. The composition according to claim 39, wherein the composition is for treating or preventing a neurological disease.

53. The composition according to claim 52, wherein the neurological disease is dementia.

54. The composition according to claim 39, wherein the composition is for treating or preventing a heart disease.

55. The composition according to claim 54, wherein the heart disease is cardiac failure.

56. The composition according to claim 54, wherein the heart disease is cardiomyopathy.

57. The composition according to claim 39, wherein the composition is for ameliorating or preventing cardiac hypertrophy.

58. The composition according to claim 39, wherein the composition is for ameliorating infertility.

59. The composition according to claim 39, wherein the composition is for activating sperm activity.

60. The composition according to claim 39, wherein the composition is for preventing formation of wrinkles in skin, for reducing a depth and/or number of wrinkles, and/or for inhibiting an increase in the depth and/or number of wrinkles.

61. The composition according to claim 39, wherein the composition is for inhibiting or preventing darkening of skin.

62. The composition according to claim 39, wherein the composition is for ameliorating or preventing obesity.

63. The composition according to claim 39, wherein the composition is for slowing progression of aging.

64. The composition according to claim 39, wherein the composition is for extending lifespan.

65. The composition according to claim 39, wherein the composition is for promoting plant growth.
